# EUROPEAN PATENT SPECIFICATION

(11) **EP 3 126 839 B1**
(45) Date of publication and mention of the grant of the patent: **12.02.2020**
(21) Application number: 15715701.7
(22) Date of filing: 30.03.2015
(51) Int. Cl.: G01N 33/536, G01N 33/543, A61K 38/17, A61K 39/395, C07K 16/44, A61K 39/00, C07K 16/18, C07K 14/47, G01N 33/68, A61K 38/00

(54) **AGENTS USEFUL IN DIAGNOSTIC AND THERAPY OF BETA-AMYLOID AGGREGATION RELATED DISORDERS AND SCREENING METHODS**
MITTEL ZUR DIAGNOSE UND THERAPIE VON MIT BETA-AMYLOID-ANHÄUFUNG VERWANDTEN ERKRANKUNGEN UND SCREENING-VERFAHREN
AGENTS UTILES DANS LE DIAGNOSTIC ET LA THÉRAPIE DES TROUBLES LIÉS À L'AGRÉGATION DE BÊTA-AMYLOÏDE ET PROCÉDÉS DE CRIBLAGE

(30) Priority: 31.03.2014 EP 14162767
(43) Date of publication of application: 08.02.2017
(73) Proprietor: Ecole Polytechnique Fédérale de Lausanne (EPFL), 1015 Lausanne (CH)
(72) Inventor: SCHMID, Adrian W., CH-1018 Lausanne (CH); MONIATTE, Marc, CH-1022 Chavannes (CH)
(74) Representative: reuteler & cie SA
(86) International application number: PCT/EP2015/056887
(87) International publication number: WO 2015/150322

(56) References cited:
- EP-A1- 2 106 802
- EP-A1- 2 149 380
- WO-A1-2010/055064
- WO-A2-2010/055510
- US-A- 5 721 106
- US-A1- 2008 274 965
- A. W. SCHMID ET AL: "Tissue Transglutaminase-mediated Glutamine Deamidation of -Amyloid Peptide Increases Peptide Solubility, Whereas Enzymatic Cross-linking and Peptide Fragmentation May Serve as Molecular Triggers for Rapid Peptide Aggregation", JOURNAL OF BIOLOGICAL CHEMISTRY, vol. 286, no. 14, 8 April 2011 (2011-04-08), pages 12172-12188, XP055142800, ISSN: 0021-9258, DOI: 10.1074/jbc.M110.176149
- TAKEKAZU KUBO ET AL: "In vivo conversion of racemized ?-amyloid ([D-Ser26]A?1-40) to truncated and toxic fragments ([D-Ser26]A?25-35/40) and fragment presence in the brains of Alzheimer's patients", JOURNAL OF NEUROSCIENCE RESEARCH, vol. 70, no. 3, 1 November 2002 (2002-11-01), pages 474-483, XP055143905, ISSN: 0360-4012, DOI: 10.1002/jnr.10391
- GORDON L M ET AL: "Structural and functional properties of peptides based on the N-terminus of HIV-1 gp41 and the C-terminus of the amyloid-beta protein", BIOCHIMICA ET BIOPHYSICA ACTA (BBA) - BIOMEMBRANES, ELSEVIER, AMSTERDAM, NL, vol. 1778, no. 10, 1 October 2008 (2008-10-01), pages 2127-2137, XP025433052, ISSN: 0005-2736, DOI: 10.1016/J.BBAMEM.2008.05.002 [retrieved on 2008-05-11]
- TANIGUCHI A., SOHMA Y. AND KISO Y.: "Click peptides as a tool for clarification of peptide- and protein functions. Prospects of their synthesis and applications seen in water-soluble precursors of amyloid-beta peptides", KAGAKU TO SEIBUTSU, vol. 48, 1 April 2010 (2010-04-01), pages 232-234, XP002730663,
- S. HEBBAR ET AL: "A fluorescent sphingolipid binding domain peptide probe interacts with sphingolipids and cholesterol-dependent raft domains", THE JOURNAL OF LIPID RESEARCH, vol. 49, no. 5, 30 January 2008 (2008-01-30), pages 1077-1089, XP055193013, ISSN: 0022-2275, DOI: 10.1194/jlr.M700543-JLR200

## Description

### Field of the Invention

The present invention relates to novel fragments of Aβ peptide found in Aβ aggregates, to molecules binding to said fragments, as well as to uses thereof as therapeutics and/or detection tools.

### Background of the Invention

Alzheimer's disease (AD) is a progressive neurodegenerative disorder that is manifested as a gradual decline in cognitive function. AD is generally characterized by amyloid deposition in senile plaques that are mainly composed of a 40- and 42- residue long amyloid β-protein (Aβ40 and Aβ42). These peptides are cleaved from the amyloid precursor protein (APP) by the sequential enzymatic activity of two proteases, β- and γ-secretases. Aβ42 plays a more important role in the pathogenesis of AD than Aβ40 because of its higher aggregation propensity and neurotoxicity *{*Haas et al, 2007, Nat. Rev. Mol. Cell. Biol. 8, 101-112*).* Several *in vitro* and *in vivo* studies have provided strong evidence that Aβ peptide-induced loss in synaptic plasticity can be associated with Aβ peptide oligomerization.

In AD, aggregation of Aβ peptide is considered a spontaneous property of this protein. However, recent reports have raised the speculation that aggregation of this protein may be strongly accelerated in the presence of tissue transglutaminase (TGase) *(*Schmid et al, 2011, J. Biol. Chem. 286, 12172-88*).* Increased TGase activity appears to coincide with AD pathology and cerebrospinal fluid (CSF) analysis of TGase levels, as well as changes in Aβ concentration have been suggested to serve as useful biomarkers in AD. Moreover, CSF analyses indicate that the presence of Aβ oligomers and the decreased levels of Aβ1-42 found in AD patients strongly correlates with AD pathology. Kubo et al., 2002, Journal of neuroscience research, 70(3), 474-483, hypothesized that a soluble Aβ peptide ([D-Ser26]API -40) is released from plagues and converted by proteolysis to toxic [D-Ser26]Aβ25-35/40, which damages hipocampal CA1 neurons by enhancing excitotoxicity in AD.

Soluble Aβ oligomers correspond to a heterogeneous protein assembly of different morphology and size and are typically referred to as low molecular weight (LMW, 8-17kDa range) oligomers of Aβ dimers, trimers and tetramers or high molecular weight (HMW, >20kDa) oligomers such as the dodecameric (56 kDa) and protofibrillar (>150 kDa) forms of Aβ. Aβ dimers and trimers have been reported to form an initial morphological entity during the early events of peptide aggregation and are therefore believed to form part of a molecular seed during oligomerization. Although the pathological loss in synaptic plasticity appears to strongly correlate with the accumulation of Aβ oligomers, the presence of Aβ dimers or trimers *per se* do not solely account for the pathological decline in neuronal plasticity. The distinct oligomeric morphology responsible for this cognitive loss and the precise mechanisms associated with this neurodegenerative process remain to be determined.

A major reason for this controversy in Aβ oligomer morphology associated neurotoxicity lies in the observation that, soluble Aβ oligomers of different morphologies may be metastable intermediate assemblies on the pathway of insoluble peptide fibrils. On the other hand, certain Aβ oligomeric assemblies may not enter a fibrillogenesis process and are therefore referred to as "off-pathway" oligomers. Nevertheless, soluble Aβ oligomers have raised the most scientific attention during the last decade, mainly because these morphologies are believed to display relatively higher neurotoxicity as compared to either monomeric Aβ or mature amyloid fibrils. Collectively, soluble Aβ oligomers meet to a certain extent the scientific criteria of being a valuable pathogenic target for the early diagnosis and treatment of AD.
WO 2010/055510 relates to methods and kits for diagnosing a subtype of mulitple sclerosis by determining the reactivity of antibodies in a sample from a subject to a plurality of antigens, various amyloid-beta peptides being part of this plurality of antigens.

The complex profile of different soluble Aβ fragments found in APP-transfected cells *(*Wang et al., 1996, Biol. Chem. 271: 31894-31902*)* or human CSF *(*Portelius et al., 2005, Neurosci. Lett. 409, 215-219*)* has been reported earlier. However, the presence of these fragments is believed to be the result of an unknown activity associated with β- and γ-secretases or, due to an undefined, concerted activity found with carboxypeptidase and metalloproteases.

Immunization against Aβ peptide is considered to be a promising approach for AD therapy because vaccination of transgenic mouse models of AD with Aβ1-42 aggregates resulted in a reduction of Aβ deposition and the prevention of cognitive impairment *(*Morgan et al, 2000, Nature 408, 982-985*).* However, clinical trials (AN1792) of immunization of AD patients against Aβ42 were interrupted because of severe adverse effects of excessive immune activation *(*Orgogozo et al, 2003, Neurology 61, 46-54*).* Recently, a follow-up study has shown that immunization against Aβ1-42 suppressed Aβ depositions in AD patients but not the progressive cognitive impairment *(*Holmes et al, Lancet 372, 216-223*).* One of the reasons for these problems might be the unintended elimination of both toxic and nontoxic forms of Aβ1-42, whose role in physiological function is controversial at present. Micelles encapsulating amyloid-beta [1-42] peptides have been developped as vaccines for amyloid-related disorders such as AD (US 2008/274965). Immunogenic peptide derivatives, in particular Aβ within the [1-28] region, have been described for the prevention and/or treatment of degenerative diseases such as AD (EP 2106802).

Antibodies binding Aβ peptide fragments are known in the art, they include, for instance, 6E10 antibody that is a mouse monoclonal antibody reactive to amino acid residues 1-16 of human Aβ peptide, the epitope lying within amino acids 3-8 of Aβ peptide (i.e. EFRHDS) *(*Perchiacca et al, 2012, PNAS, 109(1): 84-9*).* Another antibody of the prior art, 4G8 that is a mouse monoclonal antibody reactive to amino acid residues 17-24 of human Aβ peptide. The epitope recognized by 4G8 antibody lies within amino acids 18-22 of Aβ peptide (i.e. VFFAE) *{Perchiacca et al, 2012, supra).* Both 6E10 and 4G8 antibodies react to the enzymatically processed peptides Aβ40, Aβ42 and Aβ43 as well as the APP precursor form. The antibodies of the prior art are for use in research, in particular in immunoblotting (WB), immunohistochemistry (IHC), immunoprecipitation (IP), and ELISA.

Other prior art antibodies against Aβ peptide include Al 1 and NAB61. Antibody Al 1 is a conformational specific antibody which binds to a general oligomeric morphology found in different proteins such as alpha-synuclein protein, prion protein and Aβ peptide *{*Kayed etal, 2010, Mol Neurodegener. 5: 57*).* This antibody recognizes a generic epitope that is formed by many different amyloidogenic sequences, which is believed to be on the basis of the similar organization found among these proteins. Similarly, NAB61 is a monoclonal antibody that preferentially recognizes a conformational epitope present in small oligomeric and higher order Aβ structures *{*Lee et al, 2005, J. Bio. Chem., 281(7): 4292-9*).* Both All and NAB61 antibodies do not bind the soluble monomeric form of Aβ peptide, which reflects the antibody's selectivity for oligomeric morphologies of unknown sequence epitope. However, a major limitation associated with these morphology specific antibodies is the lack of specificity and sensitivity found in the initial events of Aβ aggregation, also known as the "lag-phase".

Therefore, there remains a need for the development of novel agents which show a high affinity and specificity for Aβ peptide in a form associated with presymptomatic stages of Alzheimer's disease or other beta-amyloid related disorders and exhibit a weak or limited affinity and/or specificity to the naturally occurring full-length monomeric form of Aβ peptide that is also present in healthy individuals, which rend them particularly suitable for preventive/therapeutic and prognosis/diagnostic applications in humans.

### Summary of the Invention

The present invention is directed towards Aβ-binding molecules as described herewith, in particular antibodies binding specifically to particular fragments of Aβ peptide, as well as methods for detecting the level of fragments of Aβ peptide associated with early stages of Aβ peptide aggregation and/or amyloid plaque formation, those being useful for the *ex-vivo* prognosis/diagnosis and/or prevention/treatment of beta-amyloid aggregates related disorders including Alzheimer's disease.

A first aspect of the invention provides an Aβ fragment-binding molecule characterized in that said molecule is an antibody or fragment thereof that specifically binds to a neo-C-terminal fragment of Aβ peptide obtained by cleavage of said Aβ peptide between two consecutive amino acids located between positions 20 and 26 on the amino acid sequence of said Aβ peptide, but does not bind to Aβ peptides of 40, 42 and/or 43 amino acids (Aβ1-40, Aβ1-42, Aβ1-43).

In another aspect, provides an Aβ-binding molecule characterized in that said molecule is an antibody or fragment thereof specifically binds to a fragment of Aβ peptide as described herewith but not to full-length Aβ peptides of 38, 40, 42 and/or 43 amino acids (Aβ1-38, Aβ1-40, Aβ1-42, Aβ1-43, respectively).

Another aspect of the invention provides a pharmaceutical composition comprising one or more of (i) an Aβ-binding molecule according to the invention, (ii) a fusion protein, (iii) a nucleic acid, (iv) a recombinant expression vector, and/or (v) a host cell, as described herewith, and at least one pharmaceutically acceptable carrier.

Another aspect of the invention is a kit comprising at least one Aβ-binding molecule as described herewith.

According to another aspect, the Aβ-binding molecules according to the invention or pharmaceutical compositions thereof are useful in the prevention and/or treatment of beta-amyloid aggregation related disorders.

According to another aspect of the invention, the Aβ binding molecules according to the invention are useful in an *ex vivo* method for detecting the level of Aβ peptide fragments associated with Aβ oligomerization and/or amyloid plaque formation, in particular starting from early stages of the process, in a biological sample as described herewith.

Another aspect of the invention provides novel isolated fragments of Aβ peptides as described herewith.

Another aspect of the invention provides an *ex vivo* method of prognosis or diagnosis of a β-amyloid aggregation related disorder from a biological sample of a subject comprising the steps of:
(a) Providing a biological sample from a subject;
(b) Bringing said biological sample into contact with at least one Aβ fragment-binding molecule according to any one of claims 1 to 7, wherein the contacting is under conditions sufficient for binding at least one of said neo-C-terminal fragment of Aβ present in said biological fluid sample to said at least one Aβ fragment-binding molecule;
(c) Washing;
(d) Detecting a signal proportional to the level of complex formed in step (b) between said at least one neo-C-terminal fragment of Aβ and said at least one Aβ fragment-binding molecule,
(e) Comparing the level of signal detected in step (d) with the level of signal detected in the same conditions with a negative control,
   wherein a level of signal detected in the subject's sample that is higher than the level of signal detected in the negative control is indicative of a β-amyloid aggregation related disorder.

Other features and advantages of the invention will be apparent from the following detailed description.

### Description of the figures

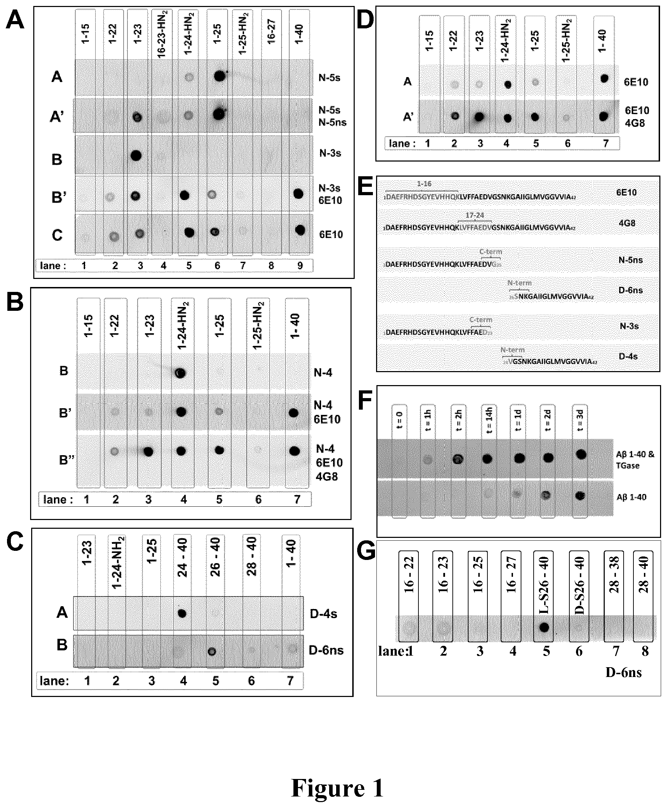

**Figure 1** **shows the epitope binding specificity of the neo-C- and neo-N-terminal antibodies using dotblotting.** Epitope binding specificity was tested using a repertoire of synthetic peptide standards of different C- or N-termini (indicated in each lane). Binding specificity of some antibodies according to the invention (N-5s, N-3s, N-5ns, N-4, D-4s, D-6ns) was compared with two commercially available anti-Aβ antibodies 6E10 and 4G8. **A:** Antibodies tested: N-5ns, N-5s, N-3s, 6E10. **B:** Antibodies tested: N-4, 6E10 and 4G8. In **A** and **B** blots A' and B' were the same as blots A and B but were re-blotted with antibody N-5ns (blot A') or 6E10 (blot B'), respectively. **C:** Antibodies tested: D-4s and D-6ns. **D:** Antibodies tested 6E10 and 4G8. E: Summary of antibody specificities. **F:** Proof of principle of binding of N-5ns antibody during Aβ 1-40 aggregation in the presence and absence of tissue transglutaminase (Tgase). (NH₂) indicates an amidated (CONH₂) form of the peptide at the C-terminal end, whereas no specific indication refers to the form of the peptide with a carboxylic acid (COOH) at the C-terminal end. **G:** Binding of D-6ns antibody to the neo-N-terminal epitope comprising L-Serine residue at the position 26 (L-S26-40; lane 5) but not to the epitope comprising Serine stereoisomer D-Serine at the position 26 (D-S26-40; lane 6).

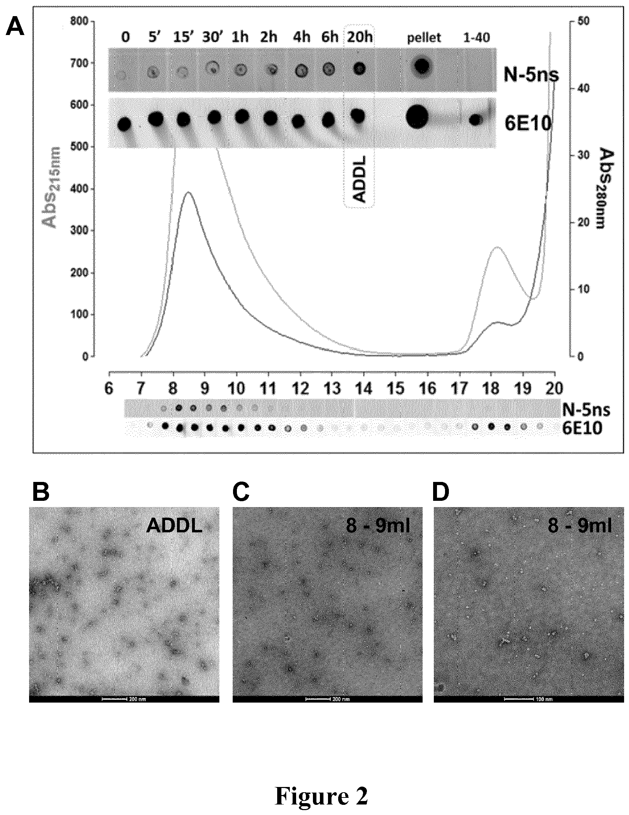

**Figure 2** **shows the screening of Aβ oligomer formation using N-5ns antibody. A:** Time dependent screening of oligomer formation and size-exclusion chromatography (SEC) of Aβ oligomers following 20 hours aggregation. **B:** Electron microscopy imaging of aggregated oligomers (ADDL's) prior to SEC. **C:** SEC purified oligomers eluted at a column volume of 8-9 ml. **D:** Zoom of purified oligomers.

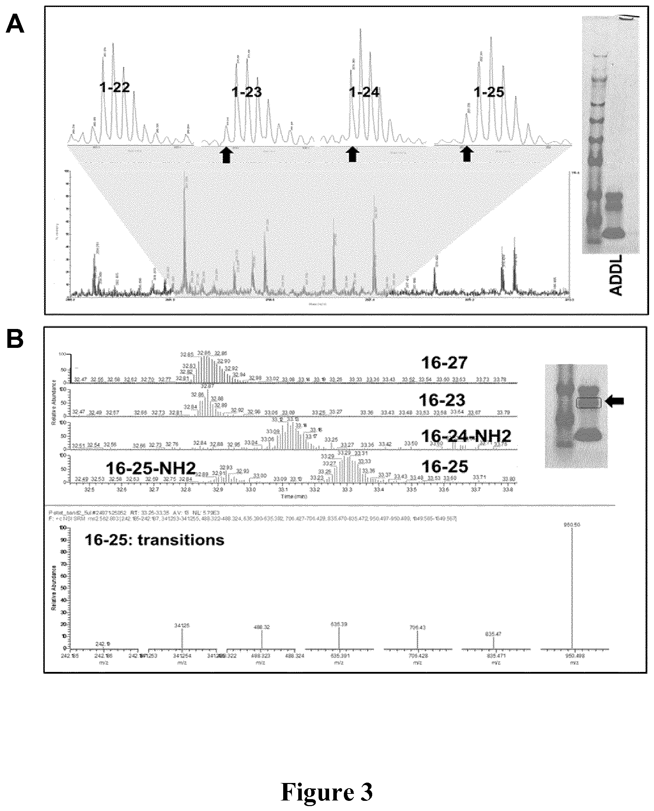

**Figure 3** **shows mass spectrometry (MALDI-TOF/TOF) analysis of Aβ oligomers and typical peptide fragment fingerprint found with oligomers. A:** MALDI-TOF/TOF analysis of oligomers. **B:** Selected reaction monitoring (SRM) mass spectrometry analysis of gel (migration height 14 kDa) extracted and Lys-N digested oligomers.

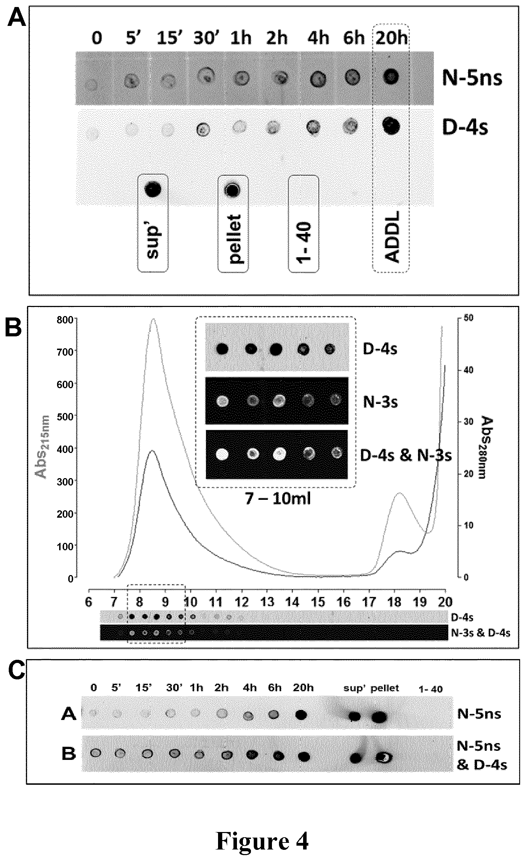

**Figure 4** **shows analysis of Aβ oligomers using the neo-N-terminal D-4s antibody and/or neo-C-terminal N-5ns/N-3s antibody. A:** Analysis of the amyloid derived diffusible ligands (ADDL's) aggregation kinetics using N-5ns or D-4s antibody. **B:** SEC analysis and dotblot analysis of oligomers using N-3s or D-4s antibody. **C:** Increased detection sensitivity with a combination of two complementary antibodies according to the invention (N-5ns and D-4s).

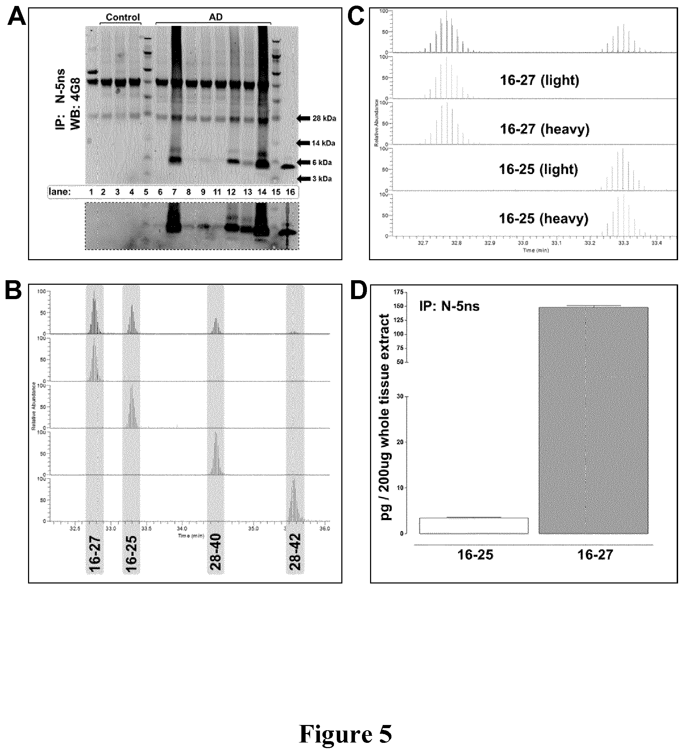

**Figure 5** **shows analysis of human control and AD patient post-mortem brain tissue extracts using immunprecipitation (IP) with N-5ns and immunoblotting. A:** Immunoblot (4G8) of human control and AD brain samples. **B-C:** Example of an analysed brain sample (IP with N-5ns followed by Lys-N digestion) using SRM, showing the identification of the diagnostic neo C-terminal fragment (16-25). **D:** SRM based quantitation of Aβ fragments in an AD brain sample.

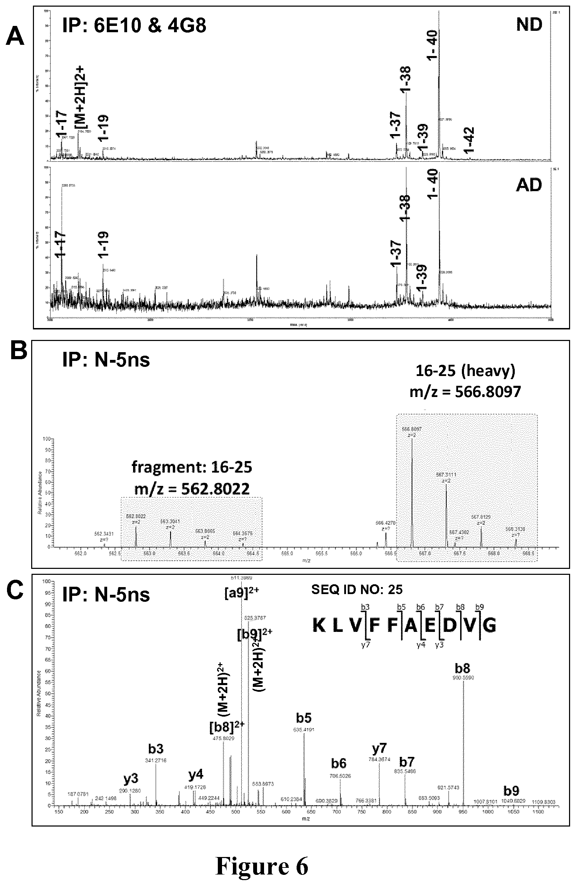

**Figure 6** **shows identification of the diagnostic neo C-terminal fragment 1-25 in human CSF sample of an AD patient. A:** IP-MS (MALDI-TOF/TOF) analysis of a control and AD CSF sample using the combination of the two commercially available antibodies 6E10 & 4G8. **B:** IP-MS analysis of an AD CSF sample using the N-5ns antibody showing the presence of the diagnostic fragment. **C:** Collision induced dissociation (CID) of the diagnostic fragment showing the identification of the peptide fragment.

### Detailed Description of the invention

The term « beta-amyloid peptide », « β-amyloid peptide », or « Aβ peptide », designates herewith peptides of 36 to 43 amino acids that are typically involved in Alzheimer's disease as the main component of the amyloid plaques found in the brains of Alzheimer patients. The Aβ peptides result from a 770-residue long amyloid precursor protein (APP, e.g. human APP, Uniprot Accession No. P05067), murine APP (Uniprot Accession No. P12023), which is cleaved before residue 672 and after residue 711, 713, or 714, by the concerted activity of β- and γ-secretases to yield Aβ1-40 (corresponding to residues 672-711 of APP), Aβ1-42 (corresponding to residues 672-713 of APP), Aβ1-43 (corresponding to residues 672-714 of APP) peptides. Aβ peptides can aggregate to form insoluble fibrils or soluble oligomers which may exist in different morphologies generally defined as low molecular weight (LMW) oligomers of Aβ dimers, trimers and tetramers or high molecular weight (HMW) oligomers such as the dodecameric (56 kDa), spherical and protofibrillar (>150 kDa) forms of Aβ. As used herewith, the term Aβ peptide includes the enzymatically cleaved peptide as defined above from the human APP and/or murine APP. For instance, the term Aβ peptide includes human Aβ peptide of amino acid sequences SEQ ID NO: 1 to SEQ ID NO: 3 corresponding to Aβ1-42, Aβ1-40, and Aβ1-43, respectively. Human and guinea pig Aβ peptide sequences are identical. It also includes murine Aβ1-43 peptide of amino acid sequence SEQ ID NO: 26, as well as murine Aβ1-40 and murine Aβ1-42, which correspond to SEQ ID NO: 26 with the last 3 amino acids at the C-terminal end of SEQ ID NO: 26 deleted, or the last amino acid at the C-terminal end of SEQ ID NO: 26 deleted, respectively. Numbers next to "Aβ" refers to the amino acid positions on the Aβ amino acid sequence.

The terms "neo-C-terminal or novo-C-terminal fragment" and "neo-N-terminal or novo-C-terminal fragment" of Aβ peptide designate herewith the two complementary fragments resulting from the intramolecular cleavage of the original Aβ peptide. Said intramolecular cleavage of Aβ peptide generates one type of peptide fragment, wherein the N-term of the original peptide remains unchanged and its C-terminal end differs from that of the original Aβ peptide ("neo-C-terminal fragment"), and a second type of peptide fragment which N-terminal end differs from that of the original Aβ peptide, while the C-terminus remain unchanged as compared to the original Aβ peptide ("neo-N-terminal fragment"). Depending on the exact position of the intramolecular cleavage, different couples of complementary fragments can be formed. Typically, the neo-C-terminal fragments and neo-N-terminal fragments of the invention result from a cleavage of the Aβ peptide between positions 20 and 26 on said Aβ peptide. The terms "neo-C-terminal fragment" and "neo-N-terminal fragment", or equal terms "novo-C-terminal fragment" and "novo-N-terminal fragment" are commonly used in the field of the invention and clear to skilled in the field of peptide chemistry in general.

The term "diagnostic peptide fragment" refers to peptide fragment of the invention that may be used for the purpose of diagnosing AD, in particular the early onsets of the disease.

An "Aβ fragment-binding molecule" as used herein relates to antibodies (including polyclonal antibodies, monoclonal antibodies, antibody fragments, antibody variants), as well as other non-antibody molecules that bind to Aβ peptide fragments as described herewith, including but not limited to antibody mimetics, portions of antibodies that mimic the structure and/or function of an antibody (Qiu et al, 2007, Nature Biotechnology 25, 921-929), chimeric proteins or fusion proteins, aptamers (including peptide aptamers, DNA and RNA aptamers), small molecules. Unless specified otherwise, an Aβ fragment-binding molecule according to the invention can bind at least one neo-C-terminal fragment of Aβ or at least one neo-N-terminal fragment of Aβ peptide as described herewith, but cannot bind full-length forms of Aβ including Aβ1-40 peptide, Aβ1-42 peptide, and Aβ1-43 peptide. According to a particular aspect of the invention, an Aβ fragment-binding molecule according to the invention can bind an Aβ peptide fragment even when incorporated within low molecular weight aggregates, such as dimeric, trimeric and tetrameric Aβ peptide species, high molecular weight oligomers, such as dodecamers, Aβ spheres, Aβ peptide protofibrils, and/or insoluble Aβ peptide fibrils, diffuse Aβ deposits, and Aβ plaques. The Aβ fragment-binding molecule according to the invention is capable of specifically binding to and, optionally, neutralizing and/or inhibiting Aβ peptide aggregation, and/or inhibiting or reducing amyloid plaque formation, and/or increasing amyloid plaque dissociation.

The term "antibody" as referred to herein designates a polypeptide that binds to an antigen. This includes whole antibodies and any antigen binding fragments. The term "antibody" is used in its broadest sense and includes monoclonal antibodies, polyclonal antibodies, human antibodies, humanized antibodies, chimeric antibodies, and further engineered antibodies as long as the characteristic properties of the invention are retained, in particular the ability of binding to the target antigen, more specifically to the same epitope of Aβ peptide fragment as the one recognized by the antibodies of the invention. Examples of antibodies and fragments thereof include a variable domain fragment ("Fv", consisting of the VH and VL domains of a single arm of an antibody), Fab fragment (monovalent fragment consisting of the VH, VL, CHI and CL domains), Fab₂ fragment (bivalent), Fab₃ fragment (trivalent), Fab' fragment (Fab with hinge region), F(ab')₂ fragment (bivalent fragment including two Fab fragments linked by a disulfide bridge at the hinge region), Fd fragment (consisting of the VH and CHI domains), rIgG (reduced IgG or half-IgG), diabodies, triabodies, tetrabodies, minibodies, domain antibodies (dAb), nanobodies, monovalent antibodies, divalent or multivalent antibodies comprising a fragment of more than one antibody, single chain variable fragment (ScFv), bis-scFv (bispecific), and derivatives of antibodies such as disulfide stabilized Fv fragments, CDR-comprising peptides, as well as epitope-binding fragments of any of the above *(*Holliger and Hudson, 2005, Nature Biotechnology, 23(9): 1126-1136*).* An antibody refers to a glycoprotein comprising at least two heavy (H) chains and two light (L) chains inter-connected by disulfide bonds, or an antigen-binding fragment thereof. Each heavy chain comprises a heavy chain variable region (VH) and a heavy chain constant region (CH). Each light chain comprises a light chain variable region (VL) and a light chain constant region (CL). In mammalians, the heavy chain can either be alpha (α), delta (δ), epsilon (ε), gamma (γ) or mu (µ), which defines the class of antibody IgA, IgD, IgE, IgG and IgM, respectively. In mammalians, the light chain can either be lambda (λ) or kappa (κ). In mammalians, depending on the class of antibody, the heavy chain constant region comprises three immunoglobulin domains, CH1, CH2, and CH3 (for IgA, IgD, IgG) or four immunoglobulin domains, CH1, CH2, CH3, and CH4 (for IgE and IgM). The light chain constant region comprises one immunoglobulin domain, CL. An antibody can have the structure of an IgA, IgG, IgE, IgD and IgM as well as any subtype thereof. Antibodies may be from any source including in particular primate (human and non-human primate) and primatized sources.

The term "variable domain" (variable domain of a light chain (VL), variable domain of a heavy chain (VH)) as used herein refers to each of the pair of light and heavy chain domains which are involved directly in binding the antibody to the antigen. The variable light and heavy chain domains have the same general structure and each domain comprises four framework ("FR") regions whose sequences are widely conserved, connected by three "hypervariable regions" called "complementary determining regions" or "CDRs". The framework regions adopt a β-sheet conformation and the CDRs may form loops connecting the β-sheet structure. The CDRs in each chain are held in their three-dimensional structure by the framework regions and form together with the CDRs from the other chain the antigen binding site. The term "antigen-binding portion of an antibody" when used herein refers to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus: the domains FR1, CDR1, FR2, CDR2, FR3, CDR3, and FR4. The residues of the CDR and FR regions are conventionally numbered according to the standard definition of Kabat et al (Sequences of Proteins of Immunological Interest, 5th ed., Public Health Service, National Institutes of Health, Bethesda, MD (1991), Publication No. 91-3242*).* The Kabat residue designations do not always correspond directly to the linear numbering of the amino acid residues. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35 (CDR- HI), residues 50-65 (CDR-H2) and residues 95-102 (CDR-H3) according to the Kabat numbering system. The CDRs of the light chain variable domain are located at residues 24-34 (CDR-L1), residues 50-56 (CDR-L2) and residues 89-97 (CDR-L3) according to the Kabat numbering system.

The term "polyclonal antibody" as used herein refers to a composition comprising different antibody molecules which are capable of binding to or reacting with several different specific antigenic determinants (also referred to herein as "epitopes") on the same or on different antigens. The variability in antigen specificity of a polyclonal antibody is located in the variable regions of the individual antibody molecules constituting the polyclonal antibody and in the particular mixture of antibody molecules that constitute the polyclonal antibody. Preferably, compositions comprising the polyclonal antibody of the invention are prepared by immunization of an animal with the target antigen or immunogenic portions thereof as specified below and are derived from the blood, milk, colostrum or eggs obtained from the immunized animal. Typically, the immunized animal is a mouse, a rabbit, or a goat.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

The term "chimeric antibody" generally refers to an antibody comprising a variable region from one source or species and at least a portion of a constant region derived from a different source or species, usually prepared by recombinant DNA techniques. A typical example of chimeric antibodies includes those comprising a murine variable region and a human constant region. As defined herewith this term also includes an antibody comprising at least one of the CDRs of a first human antibody and at least a portion of a constant region of a second human antibody. It also includes an antibody comprising heavy chain CDR1, CDR2, and CDR3 of a first human antibody and light chain CDR1, CDR2, and CDR3 of a second human antibody.

The term "humanized antibody" designates antibodies from a non-human species having one or more complementarity determining regions (CDRs) from said non-human species and a framework region from a human immunoglobulin molecule. Humanized antibodies may optionally further comprise one or more framework residues derived from the non-human species from which the CDRs were derived.

The term "human antibody" or "fully human antibody" refers to antibodies in which the variable regions and the constant regions of both the heavy and the light chains are all of human origin, or substantially identical to sequences of human origin, but not necessarily from the same antibody.

The term "isolated antibody" refers to an antibody that has been separated from a component of its natural environment. For instance, an isolated antibody has been purified to greater than 95% or 99% purity as determined by methods in the art (see e.g. Flatman et al, 2007, J Chromatogr B Analyt Technol Biomed Life Sci, 848: 79-87) including electrophoretic (e.g. SDS-PAGE, isoelectric focusing, capillary electrophoresis) or chromatographic (e.g. ion exchange or reverse phase HPLC (high performance liquid chromatography) methods.

The term "epitope" includes any polypeptide determinant capable of specific binding to an antibody. In certain embodiments, epitope determinant includes chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody. By generalization, an epitope also designates herewith a region of an antigen that is bound by an antigen binding molecule such as an Aβ fragment-binding molecule as defined herewith.

The terms "immunogenic portion" or "immunogenic fragment" of an antigen or immunogen is any portion of the antigen or immunogen that is capable of inducing an immune response in the host animal being immunized with the antigen or immunogen and that preferably causes the animal to generate polyclonal antibodies against the target antigen. In a preferred embodiment the immunogen is a neo-N-terminal or neo-C-terminal fragment of Aβ peptide as described herewith or an immunogenic fragment thereof. According to a particular aspect of the invention, the immunogenic fragment is a neo-N-terminal or neo-C-terminal fragment of Aβ peptide of synthetic origin as described herewith or an immunogenic fragment thereof.

The term "aptamer" covers peptide aptamers and nucleic acid aptamers, including polypeptidic molecules and non-coding nucleic acid molecules which bind with high specificity and affinity to the neo-N- or neo-C-termini fragments of Aβ peptides as described herewith, by adopting a specific secondary and tertiary structure. Nucleic acid aptamers may be selected from a group of sequences identified using SELEX™ (Systematic Evolution of Ligands by EXponential enrichment) method or a similar process (Cox et al., 1998, Biotechnol. Prog., 14, 845-850*;* Berezovski et al., 2006, J. Am. Chem. Soc., 128:, 1410-1411). For example, aptamers are generated via an iterative process of binding, partitioning, and amplification. Single-stranded DNA primers and templates are amplified into double-stranded transcribable templates by PCR. The library of sequences is either directly used for the selection of DNA aptamers, or transcribed to an RNA library for the selection of RNA aptamers. RNA molecules so generated are then screened for their ability to interact with neo-N- or neo-C-termini fragments of human and/or murine Aβ peptides, for example by affinity chromatography, magnetic bids, or filtration. Alternative rounds of selection and amplification are repeated under increasing stringency in order to generate a limited subset of highly specific, high-affinity candidates to the target molecule. Selected candidates may then be truncated and chemically modified to improve resistance to nucleases and pharmacological properties. Aptamers may comprise biostable aptamers, such as spiegelmers™ *(*Klussmann et al., 1996, Nat. Biotechnol., 14, 1112-1115*;* Vater and Klussmann, 2003, Curr. Opin. Drug. Discov. Devel., 6, 253-261). Peptide aptamers can be selected via *in vitro* methods such as phage display, ribosome display, and mRNA display, utilizing several rounds of peptide enrichment through *in vitro* binding to the protein target. Selected binders stay attached to the phage particle, ribosome, or mRNA molecule correspondingly, permitting the recovery of sequence information. Alternatively, peptide aptamers can also be selected via *in vivo* methods such as the Yeast two Hybrid and similar techniques. The aptamers described herein are capable of specifically binding to and, optionally, neutralizing and/or inhibiting Aβ peptide aggregation, and/or inhibiting or reducing amyloid plaque formation, and/or increasing amyloid plaque dissociation.

The terms "polynucleotide" or "nucleic acid molecule" refers to a polymer comprising nucleotides. Examples of nucleic acid molecules include DNA, RNA, locked nucleic acid (LNA), complementary DNA (cDNA).

"Polypeptide" is understood as a peptide, an oligopeptide, an oligomer or a protein comprising at least two amino acids joined to each other by a normal or modified peptide bond, such as in the cases of the isosteric peptides, for example. A polypeptide can be composed of amino acids other than the 20 amino acids defined by the genetic code. A polypeptide can equally be composed of amino acids modified by natural processes, such as post-translational maturation processes or by chemical processes, which are well known to a person skilled in the art. Such modifications are fully detailed in the literature. These modifications can appear anywhere in the polypeptide: in the peptide skeleton, in the lateral chain or even at the carboxy- or amino- terminal ends. For example, polypeptide modifications is understood to include acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bond formation, demethylation, cysteine formation, pyroglutamate formation, formylation, gamma-carboxylation, glycosylation including pegylation, GPI anchor formation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature (Proteins Structure and Molecular Properties (1993) 2nd Ed., T. E. Creighton, New York*;* Post-translational Covalent Modifications of Proteins (1983) B. C. Johnson, Ed., Academic Press, New York*;* Seifter et al. (1990) Analysis for protein modifications and nonprotein cofactors, Meth. Enzymol. 182: 626-646 *and* Rattan et al., (1992) Protein Synthesis: Post-translational Modifications and Aging, Ann NY Acad Sci, 663: 48-62)*.*

"Isolated polynucleotide" or "isolated polypeptide" is understood as a polynucleotide or a polypeptide such as previously defined which is isolated from the human body or otherwise produced by a technical process.

The term "variant" can apply to a polynucleotide and/or a polypeptide. For instance, a variant of a peptide or polypeptide, as referred to herein means a peptide or polypeptide substantially homologous to the referenced peptide sequence, but which has an amino acid sequence different from that of the referenced sequence because of one or more amino acid deletions, insertions and/or substitutions. Substantially homologous means a variant amino acid sequence which is identical to the referenced peptide sequence except for the deletion, insertion and/or substitution of a few amino acids, e.g. 1, 2, 3, 4, 5, or 6 amino acids. Substantially homologous means a variant amino acid sequence that is at least 80%, at least 85%, at least 90%, at least 95%, at least 96%, at least 97%, at least 98% or at least 99% identical to the referenced amino acid sequence. A variant nucleic acid sequence can be at least 80%, at least 85%, at least 90%, at least 95%, at least 96%, at least 97%, at least 98% or at least 99% identical to the referenced nucleic acid sequence. The identity of two amino acid sequences or of two nucleic acid sequences can be determined by visual inspection and/or mathematical calculation, or more easily by comparing sequence information using known computer program used for sequence comparison such as Clustal package version 1.83. A variant may comprise a sequence having at least one conservatively substituted amino acid, meaning that a given amino acid residue is replaced by a residue having similar physiochemical characteristics. Generally, substitutions for one or more amino acids present in the original polypeptide should be made conservatively. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known (Kyte, et al, 1982, J. Mol. Biol., 157: 105- 131)*.* For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. The term "variant" also includes a peptide or polypeptide substantially homologous to the referenced peptide sequence, but which has an amino acid sequence different from that of the referenced sequence because one or more amino acids have been chemically modified or substituted by amino acids analogs. This term also includes glycosylated polypeptides. As used herewith the term "bind" or "binding" of an antibody, or generally of an Aβ fragment-binding molecule, to a target antigen means an at least temporary interaction or association of said antibody or molecule with, or to, said target antigen (such as a neo-C-terminal fragment of Aβ peptide or a neo-N-terminal fragment of Aβ peptide as described herewith) or with, or to, fragments of said target antigen comprising an epitope recognized by said antibody or molecule.

The terms "selectively binds", "specifically binds", "specific for", when applied to an antibody or generally to an Aβ fragment-binding molecule, indicate that the antibody or molecule, preferentially recognizes and/or binds the target polypeptide or epitope, i.e. with a higher affinity than to any other antigen or epitope, i.e. the binding to the target polypeptide can be discriminated from non-specific binding to other antigens. The binding affinity of an antibody or Aβ fragment-binding molecule can be readily determined by one of ordinary skill in the art, for example, by Scatchard analysis *(*Scatchard et al., 1949, Ann. N.Y. Acad. 1949. 51, 660-672).

As used herein, "binding affinity" generally refers to the apparent association constant or "Ka". The Ka is the reciprocal of the dissociation constant "Kd". Binding affinity may be determined by a variety of methods including equilibrium dialysis, equilibrium binding, gel filtration, ELISA, surface plasmon resonance or spectroscopy (e.g. using a fluorescence assay). Exemplary conditions for evaluating binding affinity are in buffer (50 mM Tris-HCl, 150 mM NaCl, 5 mM CaCl₂ at pH 7.5). These techniques can be used to measure the concentrations of bound and free binding protein as a function of binding protein (or target) concentration. The concentration of bound binding protein ([Bound]) is related to the concentration of free binding protein ([Free]) and the concentration of binding sites for the binding protein on the target where (N) is the number of binding sites per target molecule by the following equation: [Bound]=N x([Free])/((1/Ka) + [Free]). Comparison of affinity between two antibodies can be established without actually determining the Ka value for each antibody, but based on a quantitative measurement of affinity (e.g. by ELISA or FACS analysis) that is proportional to Ka or a qualitative measurement of affinity or an inference of affinity (e.g. in functional assay or *in vitro* or *in vivo* assay).

The term "blocking" or "neutralizing" activity of an antibody, or generally of an Aβ fragment-binding molecule, refers to its ability to inhibit its target's activity. Applied to an antibody, or generally to an Aβ fragment-binding molecule, binding to as a neo-C-terminal fragment of Aβ peptide or a neo-N-terminal fragment of Aβ peptide as described herewith, this term refers to the antibody's or molecule's ability to generally inhibit or reduce Aβ peptide aggregation and/or amyloid plaque formation and/or increase amyloid plaque dissociation. The neutralizing activity of an antibody, or generally of an Aβ fragment-binding molecule, may be determined by *in vitro* assays including Thioflavin T or S fluorospectroscopy, Congo red assays, ELISA, immunoblotting, immunohistochemistry or electron microscopy. The "potency" of an antibody, or generally of an Aβ fragment-binding molecule, may be expressed as the concentration of antibody (or molecule)/antigen-binding fragment which produces the half-maximal effect at a given antigen concentration. For example, the "effect" of an antibody may be inhibition or neutralization of its target's activity. In this case, the antibody concentration producing the half-maximal inhibition may be referred to as IC₅₀, which is given in mol/l or M. Potency is usually influenced by affinity until, at a given antigen concentration, an affinity is reached beyond which further improvements in affinity will not enhance binding of the antigen anymore (so-called potency ceiling). The term "efficacy of inhibition" or "efficacy of neutralization", applied to a neutralizing antibody, or generally to an Aβ fragment-binding molecule, is a measure of effectiveness of said antibody or molecule in inhibiting a specific biological or biochemical function. Total inhibition of the biological or biochemical activity is normalized to 100%.

The term "pharmaceutically acceptable" refers to a carrier comprised of a material that is not biologically or otherwise undesirable.

The term "carrier" refers to any components present in a pharmaceutical formulation other than the active agent and thus includes diluents, binders, lubricants, disintegrants, fillers, coloring agents, wetting or emulsifying agents, pH buffering agents, preservatives and the like.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject who may be predisposed to the disease but has not yet been diagnosed as having it for example based on familial history; (b) inhibiting the disease, i.e., arresting its development; or (c) relieving the disease, i.e., causing regression of the disease and/or its symptoms or conditions such as improvement or remediation of damage. For instance, treatment of Alzheimer disease comprises preventing, decreasing or even eradicating the symptoms of the disease, for instance partial or total alleviation of cognitive decline including short term memory loss, confusion, irritability, aggression, mood swings, trouble with language, and long-term memory loss.

The terms "beta-amyloid aggregation related disorders" or "β-amyloid aggregation related disorders" are generally defined herewith as disorders associated with Aβ peptide oligomerization, aggregation, and/or amyloid plaque deposit. Non-limitative examples of β-amyloid aggregation related disorders include Alzheimer's disease (also known as Alzheimer disease), cerebral amyloid angiopathy (also known as congophilic angiopathy), Lewy body dementia (DLB), Frontotemporal dementia (FTD), inclusion body myositis, mild cognitive impairment, and β-amyloid deposition associated with aging.

Although the mechanism of Aβ peptide aggregation remain to be elucidated, the process of formation of Aβ peptides and amyloid plaque can be described as a continuous process comprising the formation of the successive following forms of amyloid beta: soluble LMW oligomers of Aβ dimers, trimers and tetramers, soluble HMW oligomers such as the dodecameric Aβ peptide species, soluble Aβ peptide protofibrils, insoluble Aβ peptide fibrils, diffuse Aβ deposits, and Aβ plaques.

"Early stage of aggregation" is defined herewith as comprising the molecular events associated with the gradual appearance of soluble LMW oligomeric Aβ peptide species. The initial molecular mechanisms associated with early stage aggregation can be considered as part of a prodromal phase of AD, because only the gradual accumulation of HMW Aβ oligomers is known to be associated with loss in neuronal plasticity and hence decline in cognitive function. In contrast, "advanced stage of aggregation" is characterized by the appearance of insoluble Aβ peptide fibrils of typical amyloidogenic nature and morphology. The advanced stage of peptide aggregation is typically monitored by fluorescence spectroscopy using molecules such as Thioflavin T and/or Thioflavin S as well as congo red. These molecules typically bind to the beta-sheet rich architecture found in amyloidogenic fibrils or large aggregates. Advanced stage of peptide aggregation can also be monitored by electron microscopy (EM) imaging. In general, fluorescence spectroscopy is not sufficiently sensitive/specific for monitoring the early stage of peptide aggregation since soluble LMW and HMW Aβ oligomers may bear little or no beta-sheet structures. The early stage of aggregation can be monitored by EM imaging or immuoblotting, which may reveal the presence of small SDS-PAGE stable oligomers. In a particular embodiment of the invention, early stage of aggregation describes the situation where soluble LMW oligomers of dimeric, trimeric and tetrameric Aβ species and soluble HMW oligomers of dodecameric, spherical and protofibrillar Aβ peptide species, as a whole, represent more than 50%, more than 60%, more than 70%, more than 80%, more than 90%, or more than 95% of the forms comprising Aβ peptide. In a more particular embodiment, "early stage of aggregation" describes the situation where soluble LMW oligomers of dimeric, trimeric and tetrameric species and soluble HMW oligomers of dodecameric, spherical and protofibrillar Aβ peptide species, as a whole, represent more than 50%, more than 60%, more than 70%, more than 80%, more than 90%, or more than 95% of the forms comprising Aβ peptide.

In another particular embodiment of the invention, early stage of aggregation describes the situation where Aβ peptide fibrils, diffuse Aβ deposits, and Aβ plaques, as a whole, represent less than 50%, less than 60%, less than 70%, less than 80%, less than 90%, or less than 95% of the forms comprising Aβ peptide.

The term "subject" as used herein refers to mammals. For example, mammals contemplated by the present invention include human, primates, domesticated animals such as cattle, sheep, pigs, horses, laboratory rodents and the like.

The term "efficacy" of a treatment or method according to the invention can be measured based on changes in the course of disease or condition in response to a use or a method according to the invention. For example, the efficacy of a treatment or method according to the invention can be measured by its impact on signs or symptoms of illness or disorder. A response is achieved when the patient experiences partial or total alleviation, or reduction of unwanted symptoms of illness or disorder.

The term "effective amount" as used herein refers to an amount of at least one agent (e.g. Aβ fragment-binding molecule) according to the invention, or a pharmaceutical formulation thereof, that elicits a detectable reduction of the symptoms of the disease in a subject that is being administered said agent, these symptoms can include, for instance: cognitive decline including short term memory loss, confusion, irritability, aggression, mood swings, trouble with language, and long-term memory loss in the case of Alzheimer's disease. When applied to a vaccine composition such as an immunogenic composition, "effective amount" designates an amount of at least one agent (e.g. neo-N-terminal or neo-C-terminal fragment of Aβ peptide from natural or synthetic origin) according to the invention, or an immunogenic formulation thereof, that elicits the production of antibodies specific for the antigen comprised in the immunogenic composition in a subject that is being administered said agent.

### Neo-N-terminal and neo-C-terminalfragments of Aβ peptide

The present invention provides fragments of Aβ peptide which may be generated as a result of aggregation mediated peptide bond hydrolysis (autocleavage), the appearance of said fragments reflecting the initiation and evolution of the Aβ aggregation process. In a particular aspect, the invention provides an isolated fragment of Aβ peptide, wherein said fragment is a neo-C-terminal fragment of Aβ peptide selected from: Aβ1-25, Aβ1-24, Aβ1-23, Aβ1-22, wherein C-terminal amino acid of said fragment is amidated.

In another particular aspect, it has been found fragments of Aβ peptide, the formation of which is associated with the early stages of Aβ aggregation which precede the appearance of the symptoms of β-amyloid aggregation related disorders, in particular Alzheimer's disease.

In one embodiment, the said fragments of Aβ peptide correspond to the neo-N-terminal and neo-C-terminal fragments of Aβ peptide obtained by cleavage of said Aβ peptide between two consecutive amino acids located between positions 20 and 26 on the amino acid sequence of Aβ peptide.

In a particular embodiment, the fragments of Aβ peptide as defined herewith are such that when the amino acid residue at the equivalent position 26 of the full-length Aβ peptide is present in said fragment, said amino acid residue is a L-Ser.

In another particular embodiment, the fragments of Aβ peptide as defined herewith comprise only L-amino acids.

In another further embodiment, the fragments of Aβ peptide as defined herewith do not include racemized Aβ fragments comprising at least one D-amino acid, in particular a D-Ser at equivalent position 26 on the full-length Aβ peptide amino acid sequence (abbreviated [D-Ser26]).

According to a more particularly embodiment, Aβ peptide fragments of sequences [D-Ser26]Ap25-40, [D-Ser26]Ap25-35, [D-Ser26]Ap26-40, [D-Ser26]Ap26-35, are not included. According to a further more particularly embodiment, Aβ peptide fragments of sequences [D-Ser26]Ap25-42, [D-Ser26]Ap25-43, [D-Ser26]Ap26-42, [D-Ser26]Ap26-43 are not included.

In a more particular embodiment, the fragments of Aβ peptide correspond to the neo-C-terminal fragments of Aβ peptide obtained by cleavage of said Aβ peptide between two consecutive amino acids present at positions 22 and 23, or 23 and 24, or 24 and 25, or 25 and 26, on the amino acid sequence of said Aβ peptide.

In another particular embodiment, the fragments of Aβ peptide correspond to the neo-C-terminal fragments of Aβ peptide obtained by cleavage of said Aβ peptide between two consecutive amino acids located between positions 22 and 23, or 23 and 24, or 25 and 26, on the amino acid sequence of said Aβ peptide.

In another particular embodiment, the fragments of Aβ peptide correspond to the neo-C-terminal fragments of Aβ peptide obtained by cleavage of said Aβ peptide between two consecutive amino acids located between positions 22 and 23, or 23 and 24, or 24 and 25, on the amino acid sequence of said Aβ peptide.

In one embodiment, said fragments comprise at least 10, at least 11, at least 12, at least 13, at least 14, at least 15, at least 16, at least 17, at least 18, at least 19, at least 20, at least 21, at least 22, or at least 23 consecutive amino acids from the full-length Aβ peptide.

In one embodiment, said fragments comprise least 24, or at least 25 consecutive amino acids from the full-length Aβ peptide.

In another embodiment, said fragments comprise not more than 30, 25, 24, 23, 22, 21, or 20 consecutive amino acids from the full-length Aβ peptide.

In the invention, said fragments may be fragments of a full-length Aβ peptide (typically Aβ1-40, Aβ1-42, or Aβ1-43) from any mammalian species including human, rabbit, rodent (mice, rat).

In one embodiment of the invention, it is provided isolated neo-C-terminal fragments of Aβ peptide, wherein said fragments correspond to Aβ1-25 or a portion thereof, in particular a portion where a few amino acids (e.g. from 1 to 12, from 1 to 5, or 1, 2, or 3) at the C-terminal end of Aβ1-25 are deleted, or a variant thereof.

In another particular aspect, the formation of fragments of Aβ peptide according to the invention is associated with the later stages of Aβ aggregation, e.g. advanced stages, which are concomitant with the development of the symptoms of β-amyloid aggregation related disorders, in particular, Alzheimer's disease.

In one embodiment, fragments of Aβ peptide are selected from the group consisting of:
(i) A neo-C-terminal fragment of Aβ peptide selected from the group consisting of:
   Aβ1-22, Aβ1-23, Aβ1-24, Aβ1-25, or a variant thereof,
(ii) A neo-C-terminal fragment of Aβ peptide selected from the group consisting of:
   Aβ1-13, Aβ1-14, Aβ1-15, or a variant thereof,

In a particular embodiment fragments of Aβ peptide are selected from the group consisting of:
(i) A neo-C-terminal fragment of Aβ peptide selected from the group consisting of:
   Aβ1-22 of SEQ ID NO: 4, Aβ1-23 of SEQ ID NO: 5, Aβ1-24 of SEQ ID NO: 6, Aβ1-25 of SEQ ID NO: 7,
(v) A neo-C-terminal fragment of Aβ peptide selected from the group consisting of:
   Aβ1-13 of SEQ ID NO: 8, Aβ1-14 of SEQ ID NO: 9, Aβ1-15 of SEQ ID NO: 10, and

In a particular embodiment of the invention, said fragment is Aβ1-23, more particularly Aβ1-23 of SEQ ID NO: 5.

In a particular embodiment of the invention, said fragment is Aβ1-24, more particularly Aβ1-24 of SEQ ID NO: 6.

In another embodiment of the invention, the last amino acid positioned at the C-terminal end of the amino acid sequence of the fragments as described above has a free -COOH group.

In an alternative embodiment of the invention, said fragments as described above are amidated at their last amino acid positioned at the C-terminal end, i.e. the last amino acid positioned at the C-terminal end of the amino acid sequence of the fragments as described above has a -CONH₂ group instead of a free -COOH group.

In a particular embodiment, an isolated fragment of Aβ peptide is a neo-C-terminal fragment of Aβ peptide selected from: Aβ1-25, Aβî-24, Aβ1-23, Aβ1-22, wherein the last C-terminal amino acid of said fragment is amidated.

In another particular embodiment of the invention, said fragment is Aβ1-24, more particularly Aβ1-24 of SEQ ID NO: 6, wherein amino acid residue at position 24 has a - CONH₂ group.

### Aβ fragment-binding molecules

The invention provides molecules specifically binding to neo-C-terminal fragments of Aβ peptide as described herewith but not binding to Aβ1-40, Aβ1-42 and/or Aβ1-43.

In a particular embodiment, the invention provides molecules specifically binding to neo-C-terminal fragments of Aβ peptide as described herewith but not binding to Aβî-40, Aβ1-42 and/or Aβ1-43.

In a particular embodiment, the invention provides Aβ fragment-binding molecule characterized in that said molecule is an antibody or a fragment thereof and specifically binds to a neo-C-terminal fragment of Aβ peptide obtained by cleavage of said Aβ peptide between two consecutive amino acids located between positions 20 and 26 on the amino acid sequence of said Aβ peptide, but does not bind to Aβ peptides of 40, 42 and/or 43 amino acids (Aβ1-40, Aβ1-42, Aβ1-43).

In one embodiment, the Aβ fragment-binding molecules bind preferentially to neo-N-terminal or neo-C-terminal fragments of Aβ peptide as described herewith and exhibit virtually no binding (i.e. negligible or not detectable binding) to Aβ1-40, Aβ1-42 and/or Aβ1-43 peptides.

In preferred embodiment, the Aβ fragment-binding molecules according to the invention bind to neo-C-terminal fragments of Aβ peptide as described herewith and exhibit virtually no binding (i.e. negligible or not detectable binding) to Aβ1-40, Aβ1-42 and/or A β1-43 peptides.

In preferred embodiment, the Aβ fragment-binding molecules according to the invention, bind to neo-C-terminal fragment of Aβ peptide obtained by cleavage of said Aβ peptide between two consecutive amino acids located at positions 24 and 25, or 23 and 24, or 25 and 26, or 22 and 23, on the amino acid sequence of said Aβ peptide.

In a particular embodiment, Aβ fragment-binding molecules bind preferentially neo-C-terminal fragments of Aβ peptide comprising only L-amino acids as described herewith and exhibit virtually no binding (i.e. negligible or not detectable binding) to Aβ1-40, Aβ1-42 and/or A β1-43 peptides.

In a particular embodiment, Aβ fragment-binding molecules exhibit virtually no binding to racemized fragments of Aβ peptide comprising at least one D-amino acid, in particular a D-Ser at equivalent position 26 relative to the amino acid sequence of full-length Aβ peptide.

In a more particular embodiment, Aβ fragment-binding molecules do not bind to racemized [D-Ser26]Aβ25-35, racemized [D-Ser26]Aβ25-40, racemized [D-Ser26]Aβ26-35, racemized [D-Ser26]Aβ26-40. In a further embodiment, Aβ fragment-binding molecules according to the invention do not bind, either, to racemized [D-Ser26]Ap25-42, racemized [D-Ser26]Ap25-43, [D-Ser26]Ap26-42 and/or racemized [D-Ser26]Ap26-43.

For therapeutic uses, it may be advantageous that the Aβ fragment-binding molecules according to the invention do not bind, and thus do not neutralize, Aβ1-40, Aβ1-42 and/or Aβ1-43 peptides so as not to substantially affect normal Aβ function.

In some embodiments, the binding affinity (e.g. inversely correlated to the Kd value) of the Aβ fragment-binding molecules for the herewith described Aβ fragments is at least 5 times, at least 10 times, at least 50 times, at least 100 times, at least 500 times, or at least 1000 times higher than their binding affinity for Aβ1-40, Aβ1-42 and Aβ1-43 peptides.

In another embodiment, Aβ fragment-binding molecules not only bind to the indicated fragments of Aβ peptide but also neutralize or inhibit and/or reduce Aβ peptide aggregation and/or amyloid plaque formation and/or increase amyloid plaque dissociation.

In one embodiment Aβ fragment-binding molecules are characterized in that said molecules specifically bind to a fragment of Aβ peptide but not to full-length Aβ peptides of 40, 42 and/or 43 amino acids (Aβ1-40, Aβ1-42, Aβî-43), wherein said fragment is a neo-C-terminal fragment of Aβ peptide obtained by cleavage of said Aβ peptide between two consecutive amino acids located between positions 20 and 26, e.g. between two consecutive amino acids present at positions 22 and 23, or 23 and 24, or 24 and 25, or 25 and 26, on the amino acid sequence of Aβ peptide.

In one embodiment of the invention, it is provided an Aβ fragment-binding molecule characterized in that said molecule specifically binds to a fragment of Aβ peptide but not to full-length Aβ peptides of 40, 42 and/or 43 amino acids (Aβ1-40, Aβ1-42, Aβî-43), wherein said fragment is neo-C-terminal fragment of Aβ peptide obtained by cleavage of said Aβ peptide between two consecutive amino acids located between positions 20 and 26, e.g. between two consecutive amino acids present at positions 22 and 23, or 23 and 24, or 24 and 25, or 25 and 26, on the amino acid sequence of Aβ peptide.

In one embodiment of the invention, it is provided an Aβ fragment-binding molecule characterized in that said molecule specifically binds to a fragment of Aβ peptide but not to full-length Aβ peptides of 40, 42 and/or 43 amino acids (Aβ1-40, Aβ1-42, Aβî-43), wherein said fragment is neo-C-terminal fragment of Aβ peptide obtained by cleavage of said Aβ peptide between two consecutive amino acids located between positions 20 and 26, e.g. between two consecutive amino acids present at positions 22 and 23, or 23 and 24, or 25 and 26, on the amino acid sequence of Aβ peptide.

In one embodiment of the invention, it is provided an Aβ fragment-binding molecule characterized in that said molecule specifically binds to a fragment of Aβ peptide but not to full-length Aβ peptides of 40, 42 and/or 43 amino acids (Aβ1-40, Aβ1-42, Aβî-43), wherein said fragment is neo-C-terminal fragment of Aβ peptide obtained by cleavage of said Aβ peptide between two consecutive amino acids located between positions 20 and 26, e.g. between two consecutive amino acids present at positions 22 and 23, or 23 and 24, or 24 and 25, on the amino acid sequence of Aβ peptide.

In one embodiment of the invention, it is provided an Aβ fragment-binding molecule characterized in that said molecule is an antibody or fragment thereof that specifically binds to a neo-C-terminal fragment of Aβ peptide obtained by cleavage of said Aβ peptide between two consecutive amino acids located between positions 20 and 26 on the amino acid sequence of said Aβ peptide, but does not bind to Aβ peptides of 40, 42 and/or 43 amino acids (Aβ1-40, Aβ1-42, Aβ1-43).

In a more particular embodiment of the invention, it is provided an Aβ fragment-binding molecule characterized in that said molecule is an antibody or fragment thereof that specifically binds to a neo-C-terminal fragment of Aβ peptide obtained by cleavage of said Aβ peptide between two consecutive amino acids located at positions 24 and 25, or 23 and 24, or 25 and 26, or 22 and 23, on the amino acid sequence of said Aβ peptide.

In one embodiment of the invention, it is provided an Aβ fragment-binding molecule characterized in that said molecule specifically binds to a fragment of Aβ peptide but not to full-length Aβ peptides of 40, 42 and/or 43 amino acids (Aβ1-40, Aβ1-42, Aβ1-43), wherein said fragment is a neo-C-terminal fragment of Aβ peptide corresponding to Aβ1-25 or a portion thereof, in particular a portion where a few amino acids, e.g. 1, 2, or 3, at the C-terminal end of Aβ1-25 are deleted, or a variant thereof.

In another embodiment Aβ fragment-binding molecules specifically bind$ to a fragment of Aβ peptide but not to Aβ peptides of 40, 42 and/or 43 amino acids (Aβ1-40, Aβ1-42, Aβî-43), wherein said fragment is a neo-N-terminal fragment of Aβ peptide corresponding to Aβ23-42 or a portion thereof, or Aβ23-40 or a portion thereof, or Aβ23-43, in particular a portion where a few amino acids, e.g. 1, 2, or 3, at the N-terminal end of Aβ23-42, Aβ23-40, and A β23-43, respectively, are deleted, or a variant thereof.

In a still other embodiment of the invention, it is provided an Aβ fragment-binding molecule characterized in that said molecule binds to a fragment of Aβ peptide but not to Aβ peptides of 40, 42 and/or 43 amino acids (Aβ1-40, Aβ1-42, Aβ1-43), wherein said fragment is a neo-C-terminal fragment of Aβ peptide corresponding to Aβ1-15 or a portion thereof, in particular a portion where a few amino acids, e.g. 1, 2, or 3, at the C-terminal end of Aβ1-15 are deleted, or a variant thereof.

In a still other embodiment Aβ fragment-binding molecules bind to a fragment of Aβ peptide but not to Aβ peptides of 40, 42 and/or 43 amino acids (Aβ1-40, Aβ1-42, Aβ1-43), wherein said fragment is a neo-N-terminal fragment of Aβ peptide corresponding to Aβ14-25 or a portion thereof, Aβ15-25 or a portion thereof, or Aβ16-25 or a portion thereof, in particular a portion where a few amino acids (e.g. from 1 to 5, or 1, 2, or 3) at the C-terminal end of Aβ14-25, Aβ15-25, and Aβ16-25, respectively, are deleted, or a variant thereof.

In a particular embodiment, the invention provides an Aβ fragment-binding molecule characterized in that said molecule specifically binds to a fragment of Aβ peptide but not to Aβ peptides of 40, 42 and/or 43 amino acids (Aβ1-40, Aβ1-42, Aβ1-43), wherein said fragment is:
(i) A neo-C-terminal fragment of Aβ peptide selected from the group consisting of:
   Aβ1-22, Aβ1-23, Aβ1-24, Aβ1-25, or a variant thereof, or
   Aβ23-43, Aβ24-43, Aβ25-43, Aβ26-43, or a variant thereof, or
(ii) A neo-C-terminal fragment of Aβ peptide selected from the group consisting of:
   Aβ1-13, Aβ1-14, Aβ1-15, or a variant thereof, or

In a particular embodiment, Aβ fragment-binding molecules specifically bind to a fragment of Aβ peptide but not to Aβ peptides of 40, 42 or 43 amino acids (Aβ1-40, Aβ1-42, Aβ1-43), wherein said fragment is:
(i) A neo-C-terminal fragment of Aβ peptide selected from the group consisting of:
   Aβ1-22 of SEQ ID NO: 4, Aβ1-23 of SEQ ID NO: 5, Aβ1-24 of SEQ ID NO: 6, A β1-25 of SEQ ID NO: 7, or
(ii) A neo-N-terminal fragment of Aβ peptide selected from the group consisting of:
   Aβ23-42 of SEQ ID NO: 11, Aβ24-42 of SEQ ID NO: 12, Aβ25-42 of SEQ ID NO: 13, Aβ26-42 of SEQ ID NO: 14, or
(iii) A neo-N-terminal fragment of Aβ peptide selected from the group consisting of:
   Aβ23-40 of SEQ ID NO: 15, Aβ24-40 of SEQ ID NO: 16, Aβ25-40 of SEQ ID NO: 17, Aβ26-40 of SEQ ID NO: 18, or
(iv)A neo-N-terminal fragment of Aβ peptide selected from the group consisting of:
   Aβ23-43 of SEQ ID NO: 19, Aβ24-43 of SEQ ID NO: 20, Aβ25-43 of SEQ ID NO: 21, Aβ26-43 of SEQ ID NO: 22, or
(v) A neo-C-terminal fragment of Aβ peptide selected from the group consisting of:
   Aβ1-13 of SEQ ID NO: 8, Aβ1-14 of SEQ ID NO: 9, Aβ1-15 of SEQ ID NO: 10, or
(vi) A neo-N-terminal fragment of Aβ peptide selected from the group consisting of:
   Aβ14-25 of SEQ ID NO: 23, Aβ15-25 of SEQ ID NO: 24, Aβ16-25 of SEQ ID NO: 25.

In a particular embodiment, said Aβ fragment-binding molecule specifically binds to a neo-C-terminal fragment of Aβ peptide selected from: Aβ1-24 and Aβ1-23.

In a particular embodiment, said Aβ fragment-binding molecule specifically binds to a neo-C-terminal fragment of Aβ peptide wherein the C-terminal amino acid is amidated.

In a particular embodiment, said Aβ fragment-binding molecule specifically binds to a fragment of Aβ peptide selected from the group consisting of: Aβ1-23 of SEQ ID NO: 5 with amino acid residue at position 23 having either a free -COOH group or is amidated (i.e. a-CONH₂ group), Aβ1-24 of SEQ ID NO: 6 with amino acid residue at position 24 having a -CONH₂ group, A β1-25 of SEQ ID NO: 7 with amino acid residue at position 25 having a -CONH₂ group.

In a more particular embodiment, said Aβ fragment-binding molecule specifically binds to a fragment of Aβ peptide that is Aβ1-24, in particular Aβ1-24 of SEQ ID NO: 6.

In a further particular embodiment, said Aβ fragment-binding molecule specifically binds to a fragment of Aβ peptide that is Aβ1-24, in particular Aβ1-24 of SEQ ID NO: 6, wherein amino acid residue at position 24 has a -CONH₂ group.

In a particular embodiment, the Aβ fragment-binding molecule according to the invention specifically binds to neo-C-terminal fragment of Aβ peptide by interacting with an epitope comprising at least one amino acid, of the Aβ peptide, selected from: A21, E22, D23, V24, and G25.

In a further particular embodiment, the Aβ fragment-binding molecule according to the invention specifically binds to said or neo-C-terminal fragment of Aβ peptide by interacting with an epitope comprising at least one amino acid, or two to three consecutive amino acids, of the Aβ peptide, selected from: A21, E22, D23, V24, and G25, wherein each one of said amino acids is a L-amino acid.

In a still further particular embodiment, Aβ fragment-binding molecules specifically bind to said neo-N-terminal or neo-C-terminal fragment of Aβ peptide by interacting with an epitope comprising at least L-S26.

It is understood that the numbers indicated herewith refer to the amino acid positions on the A β peptide sequence.

In a further particular embodiment, the Aβ fragment-binding molecule according to the invention specifically binds to said neo-C-terminal fragment of Aβ peptide by interacting with an epitope comprising at least one of the combinations of amino acids of the Aβ peptide selected from: 23DVG25, 22EDV24, and/or 21AED23,.

In a particular embodiment, the Aβ fragment-binding molecules according to the invention specifically bind to said neo-C-terminal fragments of Aβ peptide by interacting with an epitope comprising at least one of the combinations of amino acids of the Aβ peptide selected from: 22EDV24, ₂₁AED₂₃, and/or 23DVG25.

In a particular embodiment, the Aβ fragment-binding molecule according to the invention specifically binds to said neo-C-terminal fragment of Aβ peptide by interacting with an epitope comprising at least one amino acid of the Aβ peptide selected from: A21, E22, D23, V24, G25, or three consecutive amino acids of the Aβ peptide selected from: 22EDV24, 21AED₂₃, and/or ₂₃DVG25-

In another particular embodiment, the Aβ fragment-binding molecules according to the invention specifically bind to said neo-C-terminal fragments of Aβ peptide by interacting with an epitope comprising at least one of the combinations of amino acids of the Aβ peptide selected from: 23DVG25, where amino acid G has a free -COOH group or a -CONH2 group, 22EDV24, where amino acid V is amidated (i.e. has a -CONH₂ group), 2iAED₂₃ where amino acid D has a free -COOH group or a -CONH₂ group, i₃HHQi₅ where amino acid Q has a free -COOH group, and ₁₁EVH₁₃ where amino acid H has a free -COOH group.

In a more particular embodiment, the Aβ fragment-binding molecules according to the invention bind to neo-C-terminal fragments of Aβ peptide by interacting with an epitope comprising at least one combination of amino acids of the Aβ peptide selected from: ₂₃DVG₂₅, ₂₂EDV₂₄, ₂iAED₂₃,.

In a particular aspect of the invention, the Aβ fragment-binding molecules according to the invention are antibodies or fragments thereof.

In a particular aspect of the invention, said Aβ fragment-binding molecules are isolated antibodies, or fragments thereof, specifically binding to neo-C-terminal fragments of Aβ peptide as described herewith, which do not bind to A β1-40, Aβ1-42 and/or Aβ1-43.

In a particular aspect of the invention, said Aβ fragment-binding molecules are isolated antibodies, or fragments thereof, specifically binding to neo-C-terminal fragments of Aβ peptide as described herewith, which do not bind to Aβ1-40, Aβ1-42 and/or Aβ1-43. The antibodies according to the invention can be monoclonal antibodies, polyclonal antibodies, human antibodies, humanized antibodies, chimeric antibodies, and further engineered antibodies as long as the characteristic properties of the invention are retained, in particular the ability of binding to the target antigen, more specifically to the same epitope of Aβ peptide fragment as the one recognized by the antibodies of the invention, and optionally the ability of neutralizing or reducing Aβ peptide aggregation and/or amyloid plaque formation and/or the ability of increasing amyloid plaque dissociation.

In one embodiment of the invention, the isolated antibody according to the invention, or fragment thereof, is a polyclonal antibody.

Examples of polyclonal antibodies according to the invention include antibodies N-3s, N-4, N-5ns, N-5s, D-4s, and D-6ns, further characterized in the example section, as well as antibodies exhibiting similar antigen binding specificity and, in particular, recognizing the same epitopes.

In another embodiment, the isolated antibody according to the invention, or fragment thereof, is a monoclonal antibody.

In a further embodiment, the isolated antibody according to the invention, or fragment thereof, is selected from the group consisting of: a murine antibody, a humanized antibody, a human antibody, and a chimeric antibody.

Antibodies can be produced by standard techniques in the field including the recovery of polyclonal antibodies from the serum of laboratory or farm animals which have been injected with the antigen of interest, the recovery of monoclonal antibodies produced by fusing antibody-secreting spleen cells from immunized mice with immortal myeloma cell to create monoclonal hybridoma cell lines that express the specific antibody in cell culture supernatant. Alternatively, recombinant antibodies can be produced in various host cells including mammalian cells, plant cells, bacteria, yeasts, which have been engineered to express said antibodies.

### Conjugates comprising an auxiliary molecule

In another aspect of the invention, the Aβ fragment-binding molecules according to the invention, e.g. isolated antibodies or antigen-binding fragment thereof, are optionally conjugated to an accessory molecule, and are then also referred to herein as "conjugated molecules, "conjugated antibodies, or "conjugated antibody fragments", depending on the nature of the Aβ fragment-binding molecule. The accessory molecule may be conjugated to the Aβ fragment-binding molecule (e.g. an antibody or antibody fragment) directly or via a spacer of suitable length for instance as described in Kellogg et al. (2011, Bioconjug Chem, 22: 717-27*).*

In one embodiment, particularly adapted for therapeutic purposes, the accessory molecule can be a therapeutic effector group such as a cytotoxic (e.g. an enzymatically active toxin of bacterial, fungal, plant or animal origin, or fragment thereof), cytostatic, or immunomodulatory agent, including radioactive groups (i.e groups comprising a radionucleide or radioisotope), or small molecules.

In a specific embodiment, the accessory molecule may be, for example, an agent active for the treatment of a β-amyloid aggregation related disorder such as, for the treatment of Alzheihmer's disease including cholinesterase inhibitors such as Galantamine, Aricept™ (donepezil HC1), Excelon™ (rivastigmine tartrate), and NMDA (N-Methyl-D-aspartate) receptor antagonists such as Memantine.

The conjugated Aβ fragment-binding molecules according to the invention (e.g. conjugated antibodies and conjugated antibody fragments) can target the drug *in vivo* to a site of disease (e.g. the cortical brain regions or limbic system of the brain, or cerebral arties affected with cerebral amyloid angiopathy) such that the conjugated auxiliary molecule can have a therapeutic effect on the site of disease.

In an alternative embodiment, particularly adapted for diagnostic purposes, the accessory molecule can be, for example, a labeling group including radioisotopes (e.g. 3H, 14C, 32P, 35S, 1251), chromogenic labels, e.g. enzymes which can be used to convert a substrate to a detectable colored (e.g. horseradish peroxidase, alkaline phosphatase, β-galactosidase) or fluorescent compound (e.g. Green Fluorescent Protein, Red Fluorescent Protein), spectroscopic labels (e.g. fluorescent labels such as fluorescein and its derivatives like FITC, Texas red, cyanine dyes, photocyan, rhodamine, or labels presenting a visible color), luminescent labels including luciferins, affinity labels which may be developed by a further compound specific for the label and allowing easy detection and quantification, or any other label used in standard ELISA.

### Nucleic acids

According to another embodiment, it is provided an isolated nucleic acid molecule encoding: (i) a neo-N-terminal or neo-C-terminal fragment of Aβ peptide as described herewith, (ii) an Aβ fragment-binding molecule, in particular an isolated antibody or antigen-binding fragment thereof, according to the invention, or (iii) a polypeptidic conjugated molecule comprising any one of (i) or (ii) according to the invention.

In a specific embodiment, it is provided an isolated nucleic acid molecule encoding: (i) neo-C-terminal fragment of Aβ peptide as described herewith, (ii) an Aβ fragment-binding molecule, in particular an isolated antibody or antigen-binding fragment thereof or an aptamer, according to the invention, or (iii) a polypeptidic conjugated molecule comprising any one of (i) or (ii) according to the invention.

The isolated nucleic acid according to the invention may be, for instance, natural DNA or RNA or a recombinant or synthetic DNA, RNA or LNA or a recombinant nucleic acid molecule comprising any of the nucleic acid molecules according to the invention either alone or in combination. In a particular embodiment, the nucleic acid molecules according to the invention are cDNA.

### Vectors and Host cells for production and purification of the polypeptides of the invention

In one embodiment, a vector encoding an Aβ fragment binding molecule comprises a nucleic acid molecule according to the invention, wherein the vector optionally comprises an expression control sequence, allowing expression in prokaryotic or eukaryotic host cells of the encoded polypeptide, operably linked to said nucleic acid molecule.

Numerous expression systems can be used, including without limitation chromosomes, episomes, and derived viruses. More particularly, the recombinant vectors used can be derived from bacterial plasmids, transposons, yeast episomes, insertion elements, yeast chromosome elements, viruses such as baculovirus, papilloma viruses such as SV40, vaccinia viruses, adenoviruses, fox pox viruses, pseudorabies viruses, retroviruses. These recombinant vectors can equally be cosmid or phagemid derivatives.

The nucleic acid sequence can be inserted in the recombinant expression vector by methods well known to a person skilled in the art such as, for example, those that are described in MOLECULAR CLONING: A LABORATORY MANUAL, Sambrook et al, 4th Ed., Cold Spring Harbor Laboratory Press, Cold Spring Harbor, N.Y., 2001.

The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.

Thus, for example, an appropriate secretion signal can be integrated in the recombinant vector so that the polypeptide, encoded by the nucleic acid molecule of the invention, will be directed towards the lumen of the endoplasmic reticulum, towards the periplasmic space, on the membrane or towards the extracellular environment. The choice of an appropriate secretion signal may facilitate subsequent protein purification. In a further embodiment, it is provided a host cell comprising a recombinant vector according to the invention.

The introduction of the recombinant vector in a host cell can be carried out according to methods that are well known to a person skilled in the art, such as those described in BASIC METHODS IN MOLECULAR BIOLOGY, Davis et al., 2nd ed., McGraw-Hill Professional Publishing, 1995, and MOLECULAR CLONING: A LABORATORY MANUAL, supra, such as transfection by calcium phosphate, transfection by DEAE dextran, transfection, microinjection, transfection by cationic lipids, electroporation, transduction or infection.

The host cell can be, for example, bacterial cells such as *E. coli* or *Streptomyces,* cells of fungi such as *Aspergillus* and yeasts such as *Saccharomyces,* insect cells, Chinese Hamster Ovary cells (CHO), C127 mouse cell line, BHK cell line of Syrian hamster cells, Human Embryonic Kidney 293 (HEK 293) cells. In a particular embodiment, the host cell is a CHO cell or a HEK 293 cell.

The host cells can be used, for example, to express a polypeptide of the invention. After purification by standard methods, the polypeptide of the invention can be used in a method described hereinafter.

For instance, when expression systems that secrete the recombinant protein are employed, the culture medium may first be concentrated using a commercially available protein concentration filter, for example, an Amicon or Millipore Pellicon ultrafiltration unit. Following the concentration step, the concentrate can be applied to a purification matrix such as a gel filtration matrix. Alternatively, an anion exchange and/or an affinity resin can be employed. The matrices can be acrylamide, agarose, dextran, cellulose or other types commonly employed in protein purification. Alternatively, a cation exchange step can be employed. Finally, one or more reversed-phase high performance liquid chromatography (RP-HPLC) steps employing hydrophobic RP-HPLC media can be employed to further purify the antibodies or fragments thereof. Some or all of the foregoing purification steps, in various combinations, are well known and can be employed to provide a substantially homogeneous recombinant protein. Recombinant protein produced in bacterial culture can be isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps. Microbial cells can be disrupted by any convenient method, including freeze-thaw cycling, sonication, mechanical disruption, or use of cell lysing agents.

In another embodiment, the invention provides a process for producing cells capable of expressing a polypeptide according to the invention, comprising genetically engineering cells with a vector or a nucleic acid according to the invention.

In another embodiment, the invention provides a process for producing antibodies or fragments thereof according to the invention comprises culturing a host cell transformed with an expression vector comprising a nucleic sequence that encodes said antibodies or fragments thereof under conditions sufficient to promote expression of said polypeptides. The antibody or fragment thereof according to the invention is then recovered from culture medium or cell extracts, depending upon the expression system employed. As known to the skilled artisan, procedures for purifying a recombinant protein will vary according to such factors as the type of host cells employed and whether or not the recombinant protein is secreted into the culture medium as described above.

### Compositions

The invention provides pharmaceutical or therapeutic agents as compositions useful for preventing and/or treating a patient, preferably a mammalian patient, and most preferably a human patient who is suffering from, or susceptible of suffering from, a β-amyloid aggregation related disorder, in particular a disorder selected from Alzheimer's disease, cerebral amyloid angiopathy, Lewy body dementia (DLB), Frontotemporal dementia (FTD), inclusion body myositis, mild cognitive impairment, and β-amyloid deposition associated with aging.

Pharmaceutical compositions comprise one or more of: (i) an Aβ fragment-binding molecule selected from an isolated antibody or fragment thereof according to the invention, (ii) a conjugate molecule comprising the the Aβ fragment-binding molecule under (i), (iii) a nucleic acid encoding a molecule under (i) or polypeptidic conjugated molecule under (ii) (iv) a recombinant expression vector comprising the nucleic acid under (iii) and/or (v) a host cell comprising the recombinant expression vector under (iv), and at least one pharmaceutically acceptable carrier.

In a particular embodiment, said pharmaceutical composition comprises at least one Aβ fragment-binding molecule specifically binding to a neo-C-terminal fragment of Aβ peptide selected from: Aβ1-22, Aβ1-23, Aβ1-24, Aβ1-25, or a variant thereof.

In a further particular embodiment, said pharmaceutical composition comprises at least one Aβ fragment-binding molecule specifically binding to a neo-C-terminal fragment of Aβ peptide selected from: Aβ1-23, Aβ1-24, or a variant thereof.

In a still more particular embodiment, said pharmaceutical composition comprises at least one Aβ fragment-binding molecule specifically binding to Aβ1-24, more particularly Aβ1-24 where the amino acid residue at position 24 has a -CONH₂ group, but not binding to Aβ peptides of 40, 42 or 43 amino acids (Aβ1-40, Aβ1-42, Aβ1-43), and at least one pharmaceutically acceptable carrier

Pharmaceutical compositions of the invention can contain one or more Aβ fragment-binding molecule, in particular antibodies specific for the described Aβ fragments or antigen-binding fragment thereof in any form described herein.

Compositions of this invention may further comprise one or more pharmaceutically acceptable additional ingredient(s) such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, freeze-dried forms, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions of this invention may be liquid formulations including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agent include, but are not limited to, sorbitol syrup, methylcellulose, glucose/sugar syrup, gelatin, hydroxyethylcellulose, carboxymethylcellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 of Remington's The Science and Practice of Pharmacy, 22nd Edition, 2012, Pharmaceutical Press and the University of the Sciences, Philadelphia College of Pharmacy*.*

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as transdermal formulations comprising aqueous or non-aqueous vehicles including, but not limited to, creams, ointments, lotions, pastes, medicated plaster, patch, or membrane.

Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

The compounds of this invention can also be administered in sustained release forms or from sustained release drug delivery systems. A description of representative sustained release materials can also be found in *Remington's Pharmaceutical Sciences.*

Injectable formulations are particularly appropriate for administering the compositions according to the invention.

In another embodiment, the invention provides a diagnostic composition comprising at least one Aβ fragment-binding molecule according to the invention, in particular an isolated antibody or antigen-binding fragment thereof or a conjugated molecule comprising said Aβ fragment-binding molecule, and optionally a diluent and/or a carrier.

In a particular embodiment, said diagnostic composition comprises at least one Aβ fragment-binding molecule binding specifically to a neo-C-terminal Aβ peptide fragment as defined herewith.

In another embodiment provided is a diagnostic composition comprising at least one Aβ fragment-binding molecule, wherein said Aβ fragment-binding molecule is characterized in that said molecule specifically binds to a neo-C-terminal fragment of Aβ peptide obtained by cleavage of said Aβ peptide between two consecutive amino acids located between positions 20 and 26 on the amino acid sequence of said Aβ peptide, but does not bind to Aβ peptides of 40, 42 and/or 43 amino acids (Aβ1-40, Aβ1-42, Aβ1-43), wherein said Aβ fragment-binding molecule is neo-C-terminal fragment of Aβ peptide that is obtained by cleavage of said Aβ peptide between two consecutive amino acids located at positions 24 and 25, or 23 and 24, or 25 and 26, or 22 and 23, on the amino acid sequence of said Aβ peptide, wherein said Aβ fragment-binding molecule is an antibody or fragment thereof, wherein said Aβ fragment-binding molecule is neo-C-terminal fragment that is Aβ1-25 or a portion thereof where 1 to 3 amino acids at the C-terminal end of Aβ1-25 are deleted, and optionally at least one further Aβ fragment-binding molecule specifically binding to a neo-N-terminal fragment being Aβ23-42 or a portion thereof where 1 to 3 amino acids at the N-terminal end of Aβ23-42 are deleted, or Aβ23-40 or a portion thereof where 1 to 3 amino acids at the N-terminal end of Aβ23-40 are deleted, or Aβ23-43 or a portion thereof where 1 to 3 amino acids at the N-terminal end of Aβ23-43 are deleted.

More particularly, said diagnosis composition comprises: (i) an Aβ fragment-binding molecule according to the invention, wherein said neo-C-terminal fragment is Aβ1-25 or a portion thereof where 1 to 3 amino acids at the C-terminal end of Aβ1-25 are deleted.

More particularly, the diagnostic composition according to the invention comprises at least one Aβ fragment-binding molecule binding specifically to neo-C-terminal fragments Aβ1-22, Aβ1-23, Aβ1-24, and/or Aβ1-25 and at least one Aβ fragment-binding molecule binding specifically to neo-N-terminal fragments Aβ23-42, Aβ24-42, Aβ25-42, and/or Aβ26-42.

More particularly, the diagnostic composition according to the invention comprises at least one Aβ fragment-binding molecule binding specifically to neo-C-terminal fragments Aβ1-22, Aβ1-23, Aβ1-24, and/or Aβ1-25.

Still more particularly, the imaging composition or diagnostic composition according to the invention comprises at least one Aβ fragment-binding molecule binding specifically to Aβ1-24 and/or Aβ1-25.

Still more particularly, the diagnostic composition according to the invention comprises at least one Aβ fragment-binding molecule binding specifically to AβI-24-CONI^ and/or Aβ1-25 as described herewith.

Still more particularly, the diagnostic composition according to the invention comprises (i) at least one Aβ fragment-binding molecule binding specifically to one of said neo-C-terminal fragments by interacting with an epitope comprising at least one amino acid, or two to three consecutive amino acids, of the Aβ peptide, selected from: A21, E22, D23, V24, and G25.

The diagnostic composition according to the invention is useful for detecting and/or monitoring Aβ peptide aggregation and/or amyloid plaque formation *in vitro,* in particular starting from early stages of the process.

### Combination

According to the invention, an Aβ fragment-binding molecule according to the invention, in particular an isolated antibody or antigen-binding fragment thereof can be administered alone or in combination with a co-agent useful in the prevention and/or treatment of a β-amyloid aggregation related disorder, for example cholinesterase inhibitors such as Galantamine, Aricept™ (donepezil HC1), Excelon™ (rivastigmine tartrate), and NMDA (N-Methyl-D-aspartate) receptor antagonists such as Memantine.

The invention encompasses the administration of an Aβ fragment-binding molecule according to the invention, in particular an isolated antibody or antigen-binding fragment thereof, wherein the Aβ fragment-binding molecule is administered to an individual prior to, simultaneously or sequentially with other therapeutic regimens or co-agents useful in the prevention and /or treatment of a β-amyloid aggregation related disorder, in a therapeutically effective amount. The Aβ fragment-binding molecules according to the invention that are administered simultaneously with said co-agents can be administered in the same or different compositions and in the same or different routes of administration.

In a particular embodiment, an Aβ fragment-binding molecule according to the invention can be administered in combination with Aricept™ (donepezil HC1) or Memantine for the treatment of subjects suffering from Alzheimer's disease.

### Mode of administration

Compositions of this invention may be administered in any manner including, but not limited to, orally, parenterally, sublingually, transdermally, rectally, transmucosally, topically, via inhalation, via buccal or intranasal administration or intra bladder, or combinations thereof.

Parenteral administration includes, but is not limited to, intravenous, intra-arterial, intraperitoneal, subcutaneous, intramuscular, intra-thecal, and intra-articular. The compositions of this invention may also be administered in the form of an implant, which allows slow release of the compositions as well as a slow controlled i.v. infusion. In a particular embodiment, an Aβ fragment-binding molecule and/or the composition according to the invention is administered systemically or locally.

In a particular embodiment, an Aβ fragment-binding molecule and/or the composition according to the invention is administered by intranasal delivery, or by subcutaneous or intravenous route.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Patients

In an embodiment, patients according to the invention are subjects suffering from a β-amyloid aggregation related disorder, for instance Alzheimer's disease, cerebral amyloid angiopathy, Lewy body dementia (DLB), Frontotemporal dementia (FTD), inclusion body myositis, mild cognitive impairment, and β-amyloid deposition associated with aging, or subjects who may be predisposed to said disorder but have not yet been diagnosed as having it.

In a particular embodiment, patients according to the invention are subjects suffering from Alzheimer's disease or who may be predisposed to said disease but who have not yet been diagnosed as having it and/or do not exhibit the symptoms of the disease yet.

### Uses and methods according to the invention

The Aβ fragment-binding molecules according to the invention, in particular isolated antibodies or antigen-binding fragments thereof, the nucleic acids, the recombinant expression vectors, the host cells, the compositions according to the invention are useful in the prognosis, diagnosis, prevention and/or treatment of β-amyloid aggregation related disorders, as well as for monitoring Aβ aggregation and/or amyloid plaque formation, in particular starting from early stages of the process.

In one embodiment is provided an Aβ fragment-binding molecule binding specifically to a neo-C-terminal Aβ peptide fragment as defined herewith useful in the prevention and/or treatment of a β-amyloid aggregation related disorder, wherein the said molecule is used in combination with at least one further Aβ fragment-binding molecule specifically binding to a neo-N-terminal fragment obtained by cleavage of said Aβ peptide between two consecutive amino acids located between positions 20 and 26 on the amino acid sequence of said Aβ peptide, but which does not bind to Aβ peptides of 40, 42 and/or 43 amino acids (Aβ1-40, Aβ1-42, Aβ1-43).

Another embodiment provides an Aβ fragment-binding molecule according to the invention, in particular an isolated antibody or antigen-binding fragment thereof useful in the prevention and/or treatment of a β-amyloid aggregation related disorder, in particular Alzheimer's disease, cerebral amyloid angiopathy, Lewy body dementia (DLB), Frontotemporal dementia (FTD), inclusion body myositis, mild cognitive impairment, and β-amyloid deposition associated with aging, more particularly Alzheimer's disease.

In one embodiment is provided a use of an Aβ fragment-binding molecule according to the invention, in particular an isolated antibody or antigen-binding fragment thereof useful for the preparation of a pharmaceutical composition for preventing and/or treating a β-amyloid aggregation related disorder, in particular Alzheimer's disease, cerebral amyloid angiopathy, Lewy body dementia (DLB), Frontotemporal dementia (FTD), inclusion body myositis, mild cognitive impairment, and β-amyloid deposition associated with aging.

In a specific embodiment is provided an Aβ fragment-binding molecule according to the invention, in particular an isolated antibody or antigen-binding fragment thereof useful for the preparation of a pharmaceutical composition for preventing and/or treating Alzheimer's disease.

According to an embodiment, the Aβ fragments according to the invention are of synthetic origin.

According to an embodiment, the Aβ fragments according to the invention are of natural origin.

Another embodiment provides neo-N-terminal and/or neo-C-terminal Aβ fragments according to the invention useful in the prevention and/or treatment of a β-amyloid aggregation related disorder, in particular Alzheimer's disease, cerebral amyloid angiopathy, Lewy body dementia (DLB), Frontotemporal dementia (FTD), inclusion body myositis, mild cognitive impairment, and β-amyloid deposition associated with aging.

In one embodiment is provided a useful neo-N-terminal and/or neo-C-terminal Aβ fragments according to the invention useful for the preparation of a composition for preventing and/or treating a β-amyloid aggregation related disorder, in particular Alzheimer's disease, cerebral amyloid angiopathy, Lewy body dementia (DLB), Frontotemporal dementia (FTD), inclusion body myositis, mild cognitive impairment, and β-amyloid deposition associated with aging.

In a specific embodiment is provided a neo-N-terminal and/or neo-C-terminal Aβ fragments according to the invention useful for the preparation of an immunogenic composition for preventing and/or treating Alzheimer's disease.

In an alternative embodiment, Aβ fragment-binding molecules according to the invention are useful for detecting neo-N-terminal and/or neo-C-terminal fragments of Aβ as described herewith associated with Aβ oligomerization and/or amyloid plaque formation, in particular starting from early stages of the process, in a biological sample comprising contacting a biological sample from a subject with an Aβ fragment-binding molecule according to the invention, in particular an isolated antibody or antigen-binding fragment thereof.

As used herewith "biological sample" refers to cells and/or tissue samples obtained from a subject, in particular from a subject suspected of, or suffering from, a β-amyloid aggregation related disorder or at high risk of developing such a disorder.

Examples of biological sample include blood, serum, plasma, cerebrospinal fluid, synovial fluid, urine, faeces, and tissue samples including cells isolated from said tissue. Tissue samples include formalin-fixed or frozen tissue sections.

In a particular embodiment, a biological sample is a blood sample or a cerebrospinal fluid sample.

Any suitable method for detection and analysis of Aβ peptide fragments can be employed, including immunodetection (Western blot, dot blot), confocal or electron microscopy imaging coupled with immunodetection, immunoprecipitation (I), IP coupled with mass spectrometry analysis, Enzyme Linked Immunoabsorbant Assays (ELISA).

In a particular embodiment, a method for detection and analysis of Aβ peptide fragments is Enzyme Linked Immunoabsorbant Assays (ELISA).

In a particular embodiment, the invention provides an *ex vivo* method for monitoring Aβ aggregation, in particular initiation, evolution and quantification of Aβ aggregates, in a biological sample, comprising the steps of:
(i) Providing a biological sample from a subject,
(ii) Reacting said biological sample with at least one Aβ fragment-binding molecule according to the invention, under conditions sufficient for binding at least one neo-N-terminal and/or neo-C-terminal fragment of Aβ as described herewith present in said biological sample to said at least one Aβ fragment-binding molecule; and
(iii) Detecting a signal proportional to the level of complex formed in step (ii) between said Aβ fragment and said Aβ fragment-binding molecule,
wherein the intensity of the signal correlates with the level of Aβ aggregation in the biological sample.

In another embodiment, it is provided an *ex vivo* method of prognosis or diagnosis of a β-amyloid aggregation related disorder from a biological sample of a subject comprising the steps of:
(a) Providing a biological sample from a subject;
(b) Bringing said biological sample into contact with at least one Aβ fragment-binding molecule according to the invention, wherein the contacting is under conditions sufficient for binding at least one neo-C-terminal fragment of Aβ as described herewith, present in said biological fluid sample, to said at least one Aβ fragment-binding molecule;
(c) Washing;
(d) Detecting a signal proportional to the level of complex formed in step (b) between said at least one neo-N-terminal and/or neo-C-terminal fragment of Aβ and said at least one Aβ fragment-binding molecule,
(e) Comparing the level of signal detected in step (d) with the level of signal detected in the same conditions with a negative control,
wherein a level of signal detected in the subject's sample that is higher than the level of signal detected in a negative control is indicative of a β-amyloid aggregation related disorder.

Appropriate negative control includes cells from a healthy subject who is not suffering from, or susceptible to, a β-amyloid aggregation related disorder.

In another embodiment, it is provided an *ex vivo* method of prognosis or diagnosis of a β-amyloid aggregation related disorder from a biological sample wherein the said biological sample is contacted with at least one further Aβ fragment-binding molecule specifically binding to a neo-N-terminal fragment obtained by cleavage of said Aβ peptide between two consecutive amino acids located between positions 20 and 26 on the amino acid sequence of said Aβ peptide, but which does not bind to Aβ peptides of 40, 42 and/or 43 amino acids (Aβ1-40, Aβ1-42, Aβ1-43), wherein said contacting is under conditions sufficient for binding at least one of said neo-N-terminal fragment of Aβ present in said biological fluid sample to said at least one further Aβ fragment-binding molecule.

More particularly, it is provided an *ex vivo* method of prognosis or diagnosis of a β-amyloid aggregation related disorder from a biological sample of a subject comprising the steps of:
(a) Providing a biological sample from a subject;
(b) Bringing said biological sample into contact with a solid matrix where at least one antibody or fragment thereof according to the invention is bound to, wherein the contacting is under conditions sufficient for binding at least one neo-C-terminal fragment of Aβ as described herewith, present in said biological fluid sample, to said at least one antibody or fragment thereof through antigen-antibody interactions;
(c) Removing any unbound Aβ fragments from the surface of said solid matrix;
(d) Detecting a signal proportional to the level of antigen-antibody complex bound to said solid matrix,
(e) Comparing the level of signal detected in step (d) with the level of signal detected in the same conditions with a negative control,
wherein a level of signal detected in the subject's sample that is higher than the level of signal detected in the negative control is indicative of a β-amyloid aggregation related disorder.

### Kit

One aspect of the invention relates to a kit comprising at least one Aβ fragment-binding molecule according to the invention, in particular an isolated antibody or fragment thereof, and/or at least one nucleic acid encoding said Aβ fragment-binding molecule, and/or at least one recombinant expression vector comprising said nucleic acid, and/or at least one host cell according to the invention, and optionally instructional material.

In a particular embodiment, the invention provides a kit for use in a method for the detection of early stages of Aβ peptide aggregation and/or amyloid plaque formation comprising at least one Aβ fragment-binding molecule according to the invention, to be coupled or already coupled to a solid matrix.

Examples of solid matrix suitable for the invention include any solid phase support suitable for carrying out an immunoassay or a method according to the invention, such as beads, microparticles, nanoparticles, tubes, fabrics or plates, films, slides, wells, formed from or coated with glass, polystyrene, polypropylene, nitrocellulose, quartz, ceramic, dextran or other materials. For example, the solid matrix is in a form of microtiter wells, such as a 96-well microtiter plate.

The fixation of the Aβ fragment-binding molecule according to the invention, in particular the isolated antibody or antigen-binding fragment thereof, to the solid matrix in a kit according to the invention may be carried out by adsorption or chemical coupling to a solid phase support. Any means known in the art for immobilizing a protein or peptide to a solid support can be used. The isolated antibody or antigen-binding fragment thereof according to the invention can be either covalently or non-covalently bound to the solid matrix by techniques such as covalent bonding via an amide or ester linkage or adsorption. Peptides can be bound using binding pairs such as biotin and avidin or antibody and antigen. After the peptides are affixed to the solid matrix, the solid matrix can be incubated with a blocking solution (containing a blocking protein such as bovine serum albumin) to reduce non-specific adsorption of antibodies in a test sample to the support surface. According to one aspect, the antibodies or fragment thereof according to the invention can be synthesized directly on the solid matrix of the kit of the invention.

According to one embodiment, when the kit comprises at least one antibody or fragment thereof according to the invention or a combination thereof for coupling to a solid matrix as solid phase support, the kit further optionally comprises coupling reagents and/or a solid matrix for performing an immunoassay.

According to another further embodiment, the kit according to the invention further comprises at least one rinsing reagent for washing unbound material before detection in order to avoid background noise detection. Typically rinsing reagents comprise standard buffers known in the art.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### EXAMPLES

### Example 1. Identification of typical Aβ peptide fragment fingerprint found in early and advanced stages of Aβ aggregation (in vitro aggregation)

An aggregation associated stacking of Aβ peptides enables a close proximity induced side chain interaction, which may favour intra- or inter-molecular nucleophilic side chain attack. This putative nucleophilic rearrangement may result in spontaneous peptide bond hydrolysis, generating a typical peptide fragment fingerprint highly associated with peptide aggregation.

### Methods:

Synthetic Aβ peptides 1-40 and 1-42 were solubilized in phosphate buffered saline (PBS) solution at a final concentration of 0.1 and 0.05 mg/ml, respectively. Samples were incubated at 37°C and left for spontaneous aggregation over a period of 0-20 hours (early aggregation), 1-5 days (intermediate aggregation), or 1-10 weeks (long-term aggregation). Samples were drawn for mass spectrometry (MS) and dotblot analysis at different time points to assess the degree of peptide aggregation.

Matrix-assisted Laser Desorption/Ionization Time-of-flight Mass Spectrometry (MALDI TOF/TOF): Aliquots (2 µl) of samples were used for MALDI TOF/TOF MS (ABI 4800 model, Applied Biosystems) measurements. Matrix solution of α-cyano-4-hydroxycinnamic acid (7 mg/ml in ACN/0.1% TFA (1:1, v/v)) was used for sample deposition. The sample (1 µl) was mixed with 1 µl of matrix solution and then 1 µl of this mixture was deposited in duplicates on the target plate and allowed to air dry. Samples were analyzed in reflectron positive mode.

Absolute quantitation of beta-amyloid using selected reaction monitoring (SRM): All samples were analysed on a TSQ-Vantage triple quadrupole mass spectrometer (Thermo Fisher Scientific). A 0.7-FWHM-resolution window for both Q1 and Q3 was set for parent- and product-ion isolation. Fragmentation of parent ions was performed in Q2 at 1.5 mTorr, using collision energies calculated with the Pinpoint software (v1.1).

Parent-ion selection was set for fully Lys-N digested peptides on the positive charged parent ions. All CID energies and transition selection were tested manually by infusion on the TSQ using synthetic peptide standards.

A mixture of accurately quantified heavy isotope labelled peptide standards (JPT Peptide Technologies, Germany) comprising of residues: 16-23, 16-23-NH2, 16-25, 16-25-NH2, 16-27, 28-38, 28-40, 28-42 and 28-43 were spiked into each tube immediately after resuspension of samples in the Lys-N digestion buffer (50 mM ammonium bicarbonate, pH 10). Following digestion, endogenous peptides (brain samples) were extracted either from gel fractions, or in solution digestions, together with the spiked surrogate heavy peptide standards. Samples were then dried using a speed vacuum and stored at -20°C before analysis. Samples were resuspended in a solution containing 20% DMSO and 10% formic acid (FA) and further diluted to 5% DMSO and 2.5% FA prior to analysis by LC-SRM.

Using mass spectrometry analysis of synthetic Aβ1-40 and Aβ1-42 peptides, it was demonstrated that the increase in abundance of fragments of residues 1-22, 1-23, 1-24 and 1-25 represents an important fraction of a typical peptide fragment fingerprint found during the early events of peptide aggregation. The aggregation induced generation of neo C-terminal fragments is consistent with the finding that peptide aggregation in heavy labelled water (H₂¹⁸O) results in the incorporation of a heavy oxygen atom (¹⁸O) at the neo C-terminal of the above mentioned fragments (data not shown). Residual C-terminal amidation was observed in some peptide fragments, of which the relative abundance accounted for approximately 10-20% of the normal carboxy C-terminal population (data not shown). Interestingly, fragment 1-24 was found to be predominantly amidated when compared to other fragments within the close peptide region.

Since Aβ peptide aggregation is a continuous process, changes in peptide fragment fingerprints were also monitored following long-term peptide aggregation according to the protocol described above.

Mass spectrometry analysis indicated that long-term aggregation results in a clear shift in peptide fragment fingerprints, giving rise to newly formed and predominant peptide fragments comprising residues Aβ1-13, Aβ1-14 and Aβ1-15. Based on the concomitant disappearance of the longer fragments of Aβ1-23, Aβ1-24 and Aβ1-25, it was concluded that these shorter peptide fragments represent additional analytical targets for monitoring Aβ peptide aggregation (Table 1, no. 8-10). However, the appearance of these N-terminal fragments may represent a more advanced stage of Aβ peptide aggregation. This conclusion is corroborated by the observation that, long-term incubation studies of a synthetic Aβ peptide fragment 1-25 results in a gradual decrease in signal intensity of the parent peptide Aβ1-25, accompanied by a concomitant increase of the shorter fragments such as fragment 1-13 (data not shown).

The neo-C-terminal and neo-N-terminal fragments resulting from the aggregation associated Aβ peptide fragmentation, as well as the full length Aβ peptide 1-42, are indicated in **Table 1.**

**Table 1**

| **SEQ ID NO:** | **peptide** | **sequence** | **name** |
|---|---|---|---|
| Full length Aβ peptide 1-42 | | | |
| 1 | Aβ peptide 1-42 | DAEFRHDSGYEVHHQKLVFFAEDVGSNKGAIIGLMVGGVVIA | Aβ1-42 |

| Neo-C-terminal fragments | | | |
|---|---|---|---|
| 7 | Aβ peptide 1-25 | DAEFRHDSGYEVHHQKLVFFAEDVG | Aβ1-25 |
| 6 | Aβ peptide 1-24 | DAEFRHDSGYEVHHQKLVFFAEDV | Aβ1-24 |
| 5 | Aβ peptide 1-23 | DAEFRHDSGYEVHHQKLVFFAED | Aβ1-23 |
| 4 | Aβ peptide 1-22 | DAEFRHDSGYEVHHQKLVFFAE | Aβ1-22 |
| 10 | Aβ peptide 1-15 | DAEFRHDSGYEVHHQ | Aβ1-15 |
| 9 | Aβ peptide 1-14 | DAEFRHDSGYEVHH | Aβ1-14 |
| 8 | Aβ peptide 1-13 | DAEFRHDSGYEVH | Aβ1-13 |

| Neo-N-terminal fragments | | | |
|---|---|---|---|
| 14 | Aβ peptide 26-42 | SNKGAIIGLMVGGVVIA | Aβ26-42 |
| 13 | Aβ peptide 25-42 | GSNKGAIIGLMVGGVVIA | Aβ25-42 |
| 12 | Aβ peptide 24-42 | VGSNKGAIIGLMVGGVVIA | Aβ24-42 |
| 11 | Aβ peptide 23-42 | DVGSNKGAIIGLMVGGVVIA | Aβ23-42 |
| 25 | Aβ peptide 16-25 | KLVFFAEDVG | Aβ16-25 |
| 24 | Aβ peptide 15-25 | QKLVFFAEDVG | Aβ15-25 |
| 23 | Aβ peptide 14-25 | HQKLVFFAEDVG | Aβ14-25 |

### Example 2. Identification of typical Aβ peptide fragments fingerprint found in a mouse model of Alzheimer disease

IP-MS analysis of transgenic mouse (Tg2576) brain tissue (formic acid extract) was carried out using the N-5ns antibody (see examples 4 and 5 below for description).

Mass spectrometry (MALDI-TOF/TOF) analysis revealed a clear identification of the typical neo-C-terminal fragments of residues 1-22, 1-23, 1-24 and 1-25. Fragment 1-24 appeared to be predominantly amidated at its C-terminal, which is an observation also made *in vitro* with the synthetic form of Aβ1-42 peptide, whereas fragments 1-22, 1-23 and 1-25 showed residual amounts of C-terminal amidation as could be seen by the isotopic distribution and monoisotopic masses measured for these peptides (not shown). Mouse (Tg2576) brain tissue was extracted with formic acid as described above. The total protein extract was purified by gel filtration and the eluted fractions were probed for Aβ peptide using dotblotting. Fraction volumes of 11.5-12.5ml were pooled and immunoprecipitated separately using either 6E10 or 4G8. MS analysis of both immunoprecipitated samples were identical and revealed the presence of the neo-C-terminal fragment Aβ1-25 as well as full-length Aβ1-40, 1-42 and 1-43 within this fraction (not shown).

These findings strongly indicate that the presence of a typical Aβ peptide fragment fingerprint of neo-C-terminal and neo-N-terminal fragments is highly associated with *in vivo* Aβ peptide aggregation in transgenic mice.

### Example 3. Production of antibodies according to the invention

Antibodies were raised in rabbits against a peptide identical to residues 20-25 (N-5ns & N-5s), 18-23 (N-3s), 19-24 (N-4), 24-29 (D-4s) and 26-31 (D-6ns) of human Aβ peptide. All rabbit polyclonal antibodies were affinity-purified against Aβ peptide with either a -COOH or -CONH₂ C-terminal end.

Antibodies specific for a neo-C-terminal fragment of Aβ peptide as described above have a name beginning with a "N", while antibodies specific for a neo-N-terminal fragment of Aβ peptide as described above have a name beginning with a "D".

### Example 4. Characterization of some antibodies according to the invention

Epitope binding specificity of various polyclonal antibodies according to the invention, prepared as described in Example 3, was tested using a repertoire of synthetic peptide standards of different C- or N-termini. Antibody binding specificity was compared with the two commercially available anti-Aβ antibodies 6E10 and 4G8, which are known to have an Aβ N-terminal epitope binding affinity for residues 1-16 (6E10) and 17-24 (4G8), respectively.

As shown in **Figure 1A****,** binding specificity of the neo-C-terminal epitope antibodies N-5s (blot A) and N-3s (blot B) according to the invention is significantly different from the binding specificity of prior art antibody 6E10 (blot C), since N-5s and N-3s do not detect full-length Aβ 1-40 peptide (lane 9). N-5s antibody showed high binding specificity for the neo-C-terminal residue Gly25 (blot A, lane 6). The N-3s antibody appears to have a high binding affinity for the neo-C-terminal residue Asp23 (blot B, lane 3) and therefore clearly distinguishes itself from N-5s regarding C-terminal specificity. Binding specificity of N-3s antibody is significantly different from the binding specificity of prior art antibody 6E10 (blot B'). The selectivity of both antibodies N-5s and N-3s were combined in a third antibody referred to as N-5ns, which revealed an epitope binding specificity comprising the C-terminal residues Gly25 and Asp23. N-5s, N-3s, and N5ns antibodies exhibit a low binding affinity for peptide fragment 1-24 with an amidated C-terminal end (blot A', lanes 3-6). As with antibodies N-5s and N-3s, no binding to full-length Aβ1-40 peptide was observed with N-5ns (blot A', lane 9). Antibodies N-5s and N-5ns do not cross-react with other C-terminal glycine residues, such as the C-terminal of Aβ 1-38 (G38) (data not shown).

High specificity was observed for the amidated C-teminal ending of peptide fragment 1-24 using antibody N-4 (**Figure 1B**). No cross-reaction was observed with full-length peptide fragment 1-40 or any other shorter peptide fragments (blot B, lines 1-9). Binding specificity of the antibodies D-4s and D-6ns according to the invention for the complementary neo-C-terminal fragments were also tested (**Figure 1C**). Antibody D-4s showed a high affinity for the N-terminal fragment comprising residue V24 (blot A, lane 4) and a low affinity for the shorter fragment Aβ26-40 (blot A, lane 5) but did not bind to fragment Aβ28-40 (blot A, lane 6) or full-length Aβ1-40 (blot A, lane 7). Antibody D-6ns binds the peptide fragment comprising a neo-N-terminal ending at residue S26 (blot B, lane 5) but does not cross-react with the neo-N-terminal ending at residue 24 or 28, or the full-length Aβ1-40 peptide (blot B, lanes 4, 6, 7).

The high affinity for full-length Aβ1-40 of prior art antibodies 6E10 and 4G8 was confirmed and shown in **Figure 1D****,** lane 9. However the specificity of these prior art antibodies for Aβ fragments had not been studied before. Prior art antibody 4G8 has a higher binding affinity for peptide fragments Aβ1-23 and Aβ1-25, when compared to 6E10 (**Figure 1B****,** blot B', B" lanes 3 and 6 and **Figure 1D****,** blot A, A', lanes 3 and 6), whereas binding to fragment Aβ1-24 with amidated C-terminal end was clearly increased with 6E10 (**Figure 1D****,** blot A, A', lane 4). Although both 6E10 and 4G8 bind fragment Aβ1-25, they do not recognize the amidated form of this fragment **(****Figure 1D****,** blot A, A', lane 6).

The diagram represented in **Figure 1E** summarizes the neo-C-terminal binding specificity found for antibodies N-5ns and N-3s as well as for the complementary neo-N-terminal fragments targeted by D-6ns and D-4s. Further comparison with putative epitopes of antibodies 6E10 and 4G8 is provided.

Tissue transglutaminase (Tgase) mediated protein cross-linking has been reported to account for the formation of insoluble lesions found in conformational diseases such as Parkinson's disease (PD) and Alzheimer's disease (AD). Several reports have suggested that Tgase may play a key role in the pathogenesis of AD. Tgase is known to induce rapid beta-amyloid peptide aggregation by forming SDS-stable dimers and neurotoxic oligomers. Aβ1-40 peptide aggregation was monitored in the presence and absence of Tgase using the N-5ns antibody. As shown in **Figure 1F****,** the dotblot analysis revealed a positive signal for peptide aggregation as early as 1 hour in presence of Tgase, whereas in the absence of Tgase this level of significance was only attained following 1day of peptide aggregation. This data clearly indicates that the aggregation associated appearance of neo C-terminal fragments, such as peptide fragment 1-25, can be monitored using the N-5ns antibody. More importantly, the use of N-5ns allows for monitoring changes in peptide aggregation speed and therefore serves as an indispensable analytical tool for the development of novel chemical compounds aimed at inhibiting Aβ peptide aggregation. **Table 2** provides an overview of targeted neo-C-terminal and neo-N-terminal peptide fragments and the names of the tested antibodies according to the invention which bind said fragments, as well as the sequence of the epitope recognized by said antibodies.

**Table 2**

| **Peptide fragment** | **Sequence of epitope target** | **C- or N-terminal target** | **antibody** |
|---|---|---|---|
| Aβ 1-25 | ₂₃DVG₂₅-COOH | C-terminal | **N-5ns & N-5s** |
| Aβ 1-25 amidated | ₂₃DVG₂₅-CONH₂ | C-terminal | - |
| Aβ 1-24 | ₂₂EDV₂₄-COOH | C-terminal | - |
| Aβ 1-24 amidated | ₂₂EDV₂₄-CONH₂ | C-terminal | **N-4** |
| Aβ 1-23 | ₂₁AED₂₃-COOH | C-terminal | **N-3s** |
| Aβ 1-23 amidated | ₂₁AED₂₃-CONH₂ | C-terminal | - |
| Aβ 26-42 | ₂₆SNK₂₈-COOH | N-terminal | **D-6ns** |
| Aβ 24-42 | ₂₄VGS₂₆-COOH | N-terminal | **D-4s** |
| Aβ 1-15 | ₁₃HHQ₁₅-COOH | C-terminal | - |
| Aβ 1-13 | ₁₁EVH₁₃-COOH | C-terminal | - |

Specificity of the neo-N-terminal antibody binding to epitopes comprising only amino acid L-stereoisomers using dotblotting was shown on **Figure 1G****.** Epitope binding specificity was tested using a repertoire of synthetic peptide standards of different N-termini (indicated in each lane). The antibody D-6ns was specific for the neo-N-terminal epitope comprising L-Serine residue at the position 26 (L-S26-40; lane 5) but not to epitope comprising Serine stereoisomer D-Serine at the same position (D-S26-40; lane 6). No D-6ns antibody binding was observed for shorter Aβ peptide fragments (lanes 1-3) or peptide fragments comprising S26 as a consecutive sequence (lane 4).

### Example 5. Screening of beta amyloid oligomers using N-5ns antibody

Amyloid (1-42) derived diffusible ligands (ADDL's) were prepared as follows: a dried Aβ1-42 peptide film was solubilized in DMSO and further diluted in phenol red free culture media to a final concentration of 0.5 mg/ml. Samples were left for spontaneous aggregation during 0-20 hours at 4°C. Analysis of peptide fragment fingerprints was carried out at different time points in order to monitor the gradual appearance of peptide fragments as a result of peptide oligomerization. The degree of peptide aggregation was monitored by either dotblotting or MS. Five microliter aliquots were taken at each time point and used for either MS and/or dotblot analysis. Typically, one microliter of sample was diluted 1:10 with double distilled water (final 0.05 mg/ml) and spotted on a nitrocellulose membrane for dotblotting or mixed with alpha-cyano matrix for MS analysis.

Peptide aggregation was monitored at different time points by dotblotting using theN-5ns and 6E10 antibodies **(****Figure 2A****).** The gradual increase in neo-epitope signal with the N-5ns antibody revealed a time dependent peptide aggregation process taking place. A significant strong antibody signal was observed after 20 hours of peptide incubation and this was highly associated with the presence of typical oligomeric peptide morphology as revealed by electron microscopy (EM) **(****Figures 2B****).** Following peptide aggregation the sample was centrifuged in order to precipitate large fibrillar species (pellet) and the supernatant containing soluble oligomers (ADDL) was purified and fractionated by size exclusion chromatography (SEC) using a Supderdex 200 column. In order to identify the SEC fractions containing the neo-epitope of interest, SEC fractions (50 µl) were dried by speed vacuum and re-suspended in 5 µl of distilled water of which 1 µl was spotted onto a nitrocellulose membrane for dotbotting. Membranes were incubated with either the N-5ns or 6E10 antibody. Using the N-5ns antibody, a large population of neo-epitope fragments were identified within the oligomeric fractions (volume 8 ml - 10 ml). This fraction contained mainly large aggregates of protofibrillar and oligomeric morphology as revealed by electron microscopy **(****Figures 2C, D****).**

This data strongly indicates that aggregation associated generation of neo-epitopes form part of stable oligomers and that these oligomers can be specifically targeted using the N-5ns antibody.

This crucial finding allows for specific prognosis/diagnostic analysis and targeted depletion of toxic oligomers *in vitro and in vivo,* using the specific neo-C-terminal and neo-N-terminal epitope antibodies described herein.

### Example 6. Selected Reaction Monitoring (SRM) mass spectrometry of gel extracted low molecular weight (LMW) oligomers

Applying a similar protocol as described in Example 1, MALDI-TOF/TOF analysis of amyloid derived diffusible ligands (ADDL's) showed the presence of the typical peptide fragment fingerprint generated during ADDL formation **Figure 3A****.** The zoom shows the monoisotopic distribution of the peptide fragments typically found during aggregation. The arrows indicate the presence of the monoisotopic peak corresponding to an amidated C-terminal peptide. Fragment Aβ1-25 and Aβ1-23 appeared to have a relative abundance of 20%-30% of C-terminal amidation, whereas fragment Aβ1-24 was predominantly observed with a fully amidated at the C-terminal. Inset: SDS-PAGE (coomassie stained) showing the gel migration pattern typically observed with these ADDL's.

SDS-PAGE gel bands were cut at the migration level of the Aβ tetramer, trimer, dimer and monomer level. Protein bands were subjected to Lys-N in gel digestion and the extracted proteolytic fragments were analyzed by SRM mass spectrometry **(****Figure 3B****,** upper panel). The total ion extraction chromatogram of the SRM analysis shows an example of the analysis of the trimer band (gel migration height of 14kDa) **(****Figure 3B****,** gel inset: arrow), where the fragment of residues 16-27 represents the abundance of full-length peptide present in the trimer band since Lys-N proteolysis cleaves full-length Aβ1-42 peptide at positions 15 and 28. Fragments 16-23, 16-24-HN₂ (amidated C-term) and 16-25 or 16-25-NH₂ (amidated C-term) represent the typical peptide fragment fingerprint found within a population of soluble oligomers and are therefore representative of the neo-C-terminal fragments associated with Aβ aggregation. A clear chromatographic separation is achieved between normal, carboxy (COOH) and amidated (CONH₂) C-terminal fragments. The window below shows the identification of the SRM peptide fragment transitions monitored for fragment 16-25 **(****Figure 3B****,** lower panel).

In summary, this data demonstrates that LMW oligomers, such as Aβ trimers and/or tetramers, as well as larger Aβ oligomeric assemblies, incorporate a substantial amount of neo-C-terminal and possibly neo-N-terminal fragments within their structure. This implies that soluble Aβ oligomers can be specifically targeted using the herein described neo-epitope antibodies such as N-5ns, N-3s, N4 or D-4s and D-6ns respectively.

### Example 7. Analysis of Abeta oligomers using the D-4s antibody

Aβ peptide aggregation was monitored using D-4s antibody according to the invention following the protocol described in Example 5.

Analysis of the ADDL's aggregation kinetics shows a similar time dependent increase in D-4s antibody signal as found with N-5ns **(****Figure 4A****).**

Dotblot (D-4s) analysis of the purified ADDL sample shows a significant signal only for the oligomeric fraction **(****Figure 4B****).** These oligomers were also detected using the neo C-terminal specific N-3s antibody, which binds to the complementary peptide fragment 1-23. No signal was observed for the monomeric fraction using either antibody.

### Example 8. The use of a combination of two complementary antibodies increases detection sensibility

Since antibodies N-5ns, N-3s and D-4s showed selective binding for fragments found within the oligomer (ADDL) fraction, it was investigated whether the detection sensibility was altered by using a combination of two complementary antibodies. An antibody mixture consisting of N-5ns and D-4s was used to monitor the aggregation kinetics of Aβ1-42 and compared with the signal readout with only one antibody (N-5ns) alone.

The results clearly demonstrate an amplification of antibody signal using a combination of two antibodies as compared to a single antibody **(****Figure 4C****).**

### Example 9. Use of N-5ns antibody for the detection of Aβ1-25 fragment and diagnosis of Alzheimer disease in humans

Human brain tissue samples were serially extracted using TBS, SDS and formic acid as described earlier. Briefly, tissue samples were homogenized (20 strokes on ice) in TBS with protease inhibitors (Roche, Switzerland) using a Teflon homogenizer. Samples were then subjected to centrifugation (150,000g) during 45min and supernatant was recovered as the TBS soluble fraction. Protein pellets were subjected to serial extractions using either *Triton-X 100* (1%) or SDS (2%) followed by centrifugation. Finally, pellets were extracted with 70% formic acid (FA) overnight at 4°C followed by centrifugation. SDS fractions were diluted to 0.1% SDS final concentration and FA fractions were neutralized to pH 7.5 with 5M NaOH solution prior to IP. All samples were initially depleted of endogenous IgG's using a mixture of protein A&G agarose (Roche, Switzerland). Typically, 3-5ug/ml rabbit polyclonal antibody (N-3s, N-5s or N-5ns) or 3-5ug/ml of 6E10 or 4G8 mouse monoclonal was used for overnight IP under continues rotation (4rpm/min) at 4°C. Following IP, samples were either directly analysed by WB, by MALDI-TOF/TOF or further digested overnight using Lys-N proteolysis prior to SRM analysis as outlined above. Human CSF samples (500 µl) were immunoprecipitated with either N-5ns, or a mixture of the two commercial antibodies 6E10 and 4G8. Human CSF samples were pre-cleared of endogenous IgG antibodies during 4hr at 4°C. Antibody N-5ns was bound to protein A agarose beads during 2hr at 4°C without crosslinking. CSF samples were IP'ed over night at 4°C under constant rotation. Following IP, samples were washed twice with IP buffer and eluted with 40% ACN/H₂O & 0.1% TFA and dried in a speed vacuum. Samples were re-suspended in 50 µl digestion buffer (50 mM ammonium bicarbonate pH 9-10) and digested overnight at 37°C with Lys-N (70 ng). Following digestion, samples were dried and re-suspended in 20-30 µl of 10% DMSO & 5% FA. prior to LC-MS analysis. Where indicated, samples were spiked with a heavy labelled (lysine, K) surrogate peptide corresponding to the Lys-N cleavage product of residues 16-27, 16-25, 16-24 and 16-23.

Immunoprecipitation (IP) using N-5ns antibody, of human control and Alzheimer's disease (AD) post-mortem brain samples resulted in a pull-down of low molecular weight oligomers in AD samples **(****Figure 5A****,** lanes 6-14). No oligomers or monomers were detected in human control brain samples **(****Figure 5A****,** lanes 2-4). These findings highlight the specificity of the N-5ns antibody for the detection of diagnostic peptide fragments with neo C-termini such as peptide fragment 1-25 found in a complex sample matrix. Moreover, IP with N-5ns allows for an identification of oligomeric Aβ species, since neo C-terminal fragments form a stable entity with HMW oligomers. SRM mass spectrometry was used for the targeted analysis of diagnostic fragments (post Lys-N digestion) and the resulting data is shown in **Figure 5B****.** The total ion chromatogram shows the liquid chromatography (LC) elution profile and MS identification of fragments 16-27 (representative of full-length Aβ digest), 16-25 (representative of fragment 1-25 digest) and the complementary C-terminal fragments of 28-40 and 28-42. The SRM findings corroborate the observation made by WB shown in **Figure 5A****,** that IP of brain samples results in a pull-down of the target peptide fragment together with full-length Aβ 1-40/42. The spiking with heavy labelled peptides standards allows for a comprehensive identification and quantitation of endogenous peptide fragments, since these surrogate peptides have identical physico-chemical properties as the light, endogenous peptides **(****Figure 5C**). Determination of absolute values for fragment 1-25 was achieved using SRM mass spectrometry and the quantitative results are shown in **Figure 5D****.** These findings indicate that IP of human brain tissue using N-5ns targets a small population (≥5 pg) of the diagnostic peptide fragment 1-25 and that these fragments form part of larger aggregates as seen by the presence of full-length Aβ peptide (16-27) (≤150 pg) **(****Figure 5D****).**

These findings further show the specificity of the N-5ns antibody for the detection of a small population of neo-C-terminus peptide fragments 1-25 found in larger peptide aggregates found in Alzheimer's disease post-mortem brain samples.

### Example 10. Immunoprecipitation (IP) of human cerebrospinal fluid (CSF) derived from a clinically diagnosed AD patient

IP-MS analysis of a human AD and non-demented (ND) control CSF sample using a combination of two commercially available antibodies (6E10 & 4G8). MS analysis (MALDI-TOF/TOF) showed no significant difference in detection of Aβ peptide fragments between these two samples **(****Figure 6A****).**

In order to reveal the presence of any diagnostic peptide fragments, CSF samples were immunoprecipitated with N-5ns antibody and subjected to by Lys-N digestion (N-terminal cleavage at residue K16) prior to targeted LC-MS analysis. Samples were spiked with a known concentration of the heavy (lysine, K) labelled surrogate peptide which represents the lys-N cleavage product of residues 16-25. **Figure 6B** shows the LC-MS analysis of a human CSF sample derived from a clinically diagnosed AD patient subjected to IP with N-5ns and LC-MS analysis, revealing the presence of the diagnostic peptide fragment 16-25 ([M+2H]²⁺ m/z= 562.8022) (post Lys-N digest) as well as the heavy labelled surrogate (spiked reference) peptide ([M+2H]²⁺ m/z= 566.8097). Collision induced dissociation (CID) allowed for comprehensive identification of the (endogenous) diagnostic peptide fragment of residues 16-25 and the fragment ions are shown in **Figure 6C****.**

These findings show the specificity and efficiency of the N-5ns antibody for the detection of peptide fragments of the invention within CSF derived from a clinically diagnosed AD patient.

### Example 11. Measurement of oligomeric Aβ in human CSF using sandwich ELISA approach

Antibodies of the invention, in particular, N-5ns antibody can be used for the detection of peptide fragments of the invention within CSF derived from a patient, as illustrated below.

CSF is known to closely reflect the AD pathology associated changes in Aβ peptide (see Example 10). Changes in CSF levels of Aβ x-42, total and phosphorylated tau, as well as the combination of total tau and Aβ x-42 levels allows to differentiate AD from normal aging and from other forms of dementia such as frontotemporal dementia (FTD). Today, CSF biomarkers for AD have reached a scientific consensus regarding the use of these specific biomarkers for differentiation of early and incipient AD from normal aging subjects. Aβ oligomers are collectively defined as a group of neuro-toxic Aβ aggregates of pre-fibrillar morphology and reflect the initial, early events of Aβ misfolding associated with AD pathology. Therefore, the presence of toxic Aβ oligomeric species found in CSF or blood of AD patients may highlight the onset of AD pathology. Targeted and quantitative MS analysis of Aβ oligomers indicates that aggregation associated auto-cleavage at positions D23, V24 or G25 results in the generation of N-terminal fragments with high aggregation properties, which in turn gives rise to the formation of large Aβ oligomers, consisting of full-length Aβ1-42 as well as novo C-terminal and N-terminal fragments **(****Figure 2** **and** **4****)**. Antibodies of the invention (N-3s, N-4, N-5s, N-5ns, optionally in combination with D-4s and D-6ns) therefore serve as an indispensable analytical and diagnostic tool for the use of capture antibodies in diagnostic ELISAs, because these antibodies target specific epitopes exposed on the surfaces both LMW and HMW Aβ oligomers.

Enzyme-linked immunosorbend assays (ELISA) are typically used for the detection of oligomeric Aβ in CSF using a "sandwich" ELISA approach. The sandwich ELISA approach involves the use of a capture anti-Aβ antibody allowing for immobilization of Aβ oligomers on the plate surface, whereas the application of a second, typically biotinylated anti-Aβ antibody (of identical or similar binding epitope) allows for the detection of any free binding surfaces present in Aβ oligomers. Hence, the larger the surface of the oligomer the stronger the signal readout of the second antibody.

Two independent standard curves are generated using synthetic, aggregated (t=3d) Aβ peptide fragment 1-25 alone, or as a peptide mixture of aggregated (t=3d) fragments: 1-23, 1-24 and 1-25 at a total peptide concentration range of 5 to 50 pg/ml in PBS. The antibody of invention N-5ns is used as a capture antibody whereas the commercially available mouse monoclonal, biotinylated form of 6E10 is used as a detection antibody. Capture antibody (N-5ns, 50 µl, 20 µg/ml) is bound to a PVC, 96 well plate following over-night incubation at 4°C to allow complete binding. Following binding, the wells are washed twice with PBS. Blocking of unbound well surfaces is done by using 3% BSA/PBS with 0.02% sodium azide and left for 2h at room temperature followed by two washing steps. CSF samples from clinically diagnosed AD patients and non-demented controls (ND) are thawed on ice. Samples and controls are added in duplicates at 75 µl per assay well. The plate is incubated overnight on a shaker at 4°C. The following day, the plate is washed three times with PBS, 0.05% Tween. The detection antibody (6E10) is added to the wells and left for incubation during 2h room temperature and washed several times with PBS prior to plate reading.

This protocol presents the method of use of the N-5ns antibody for the detection of peptide fragments within CSF derived from a patient.

### LIST OF SEQUENCES

SEQ ID NO: 1: human Aβ1-42
   DAEFRHDSGYEVHHQKLVFFAEDVGSNKGAIIGLMVGGVVIA
SEQ ID NO: 2: human Aβ1-40
   DAEFRHDSGYEVHHQKLVFFAEDVGSNKGAIIGLMVGGVV
SEQ ID NO: 3: human Aβ1-43
   DAEFRHDSGYEVHHQKLVFFAEDVGSNKGAIIGLMVGGVVIAT
SEQ ID NO: 4: Aβ1-22
   DAEFRHDSGYEVHHQKLVFFAE
SEQ ID NO: 5: Aβ1-23
   DAEFRHDSGYEVHHQKLVFFAED
SEQ ID NO: 6: Aβ1-24
   DAEFRHDSGYEVHHQKLVFFAEDV
SEQ ID NO: 7: Aβ1-25
   DAEFRHDSGYEVHHQKLVFFAEDVG
SEQ ID NO: 8: Aβ1-13
   DAEFRHDSGYEVH
SEQ ID NO: 9: Aβ1-14
   DAEFRHDSGYEVHH
SEQ ID NO: 10: Aβ1-15
   DAEFRHDSGYEVHHQ
SEQ ID NO: 11: Aβ23-42
   DVGSNKGAIIGLMVGGVV
SEQ ID NO: 12: Aβ24-42
   VGSNKGAIIGLMVGGVVIA
SEQ ID NO: 13: Aβ25-42
   GSNKGAIIGLMVGGVVIA
SEQ ID NO: 14: Aβ26-42
   SNKGAIIGLMVGGVVIA
SEQ ID NO: 15: Aβ23-40
   DVGSNKGAIIGLMVGGVV
SEQ ID NO: 16: Aβ 24-40
   VGSNKGAIIGLMVGGVV
SEQ ID NO: 17: Aβ25-40
   GSNKGAIIGLMVGGVV
SEQ ID NO: 18: Aβ26-40
   SNKGAIIGLMVGGVV
SEQ ID NO: 19: Aβ23-43
   DVGSNKGAIIGLMVGGVVIAT
SEQ ID NO: 20: Aβ24-43
   VGSNKGAIIGLMVGGVVIAT
SEQ ID NO: 21: Aβ25-43
   GSNKGAIIGLMVGGVVIAT
SEQ ID NO: 22: Aβ26-43
   SNKGAIIGLMVGGVVIAT
SEQ ID NO: 23: Aβ14-25
   HQKLVFFAEDVG
SEQ ID NO: 24: Aβ15-25
   QKLVFFAEDVG
SEQ ID NO: 25: Aβ16-25
   KLVFFAEDVG
SEQ ID NO: 26: Mouse Aβ1-43:
   DAEFGHDSGFEVRHQKLVFFAEDVGSNKGAIIGLMVGGVVIAT

## Claims

1. An Aβ fragment-binding molecule **characterized in that** said molecule is an antibody or fragment thereof that specifically binds to a neo-C-terminal fragment of Aβ peptide obtained by cleavage of said Aβ peptide between two consecutive amino acids located between positions 20 and 26 on the amino acid sequence of said Aβ peptide, but does not bind to Aβ peptides of 40, 42 and/or 43 amino acids (Aβ1-40, Aβ1-42, Aβ1-43).

2. An Aβ fragment-binding molecule according to claim 1, wherein said neo-C-terminal fragment of Aβ peptide is obtained by cleavage of said Aβ peptide between two consecutive amino acids located at positions 24 and 25, or 23 and 24, or 25 and 26, or 22 and 23, on the amino acid sequence of said Aβ peptide.

3. An Aβ fragment-binding molecule according to any one of claims 1 to 2, wherein said neo-C-terminal fragment is Aβ1-25 or a portion thereof where 1 to 3 amino acids at the C-terminal end of Aβ1-25 are deleted.

4. The Aβ fragment-binding molecule according to claim 3, wherein said neo-C-terminal fragment is selected from: Aβ1-24 and Aβ1-23.

5. The Aβ fragment-binding molecule according to any one of claims 1 to 4, wherein said Aβ fragment-binding molecule specifically binds to said neo-C-terminal fragment of Aβ peptide by interacting with an epitope comprising at least one amino acid of the Aβ peptide selected from: A21, E22, D23, V24, G25, or three consecutive amino acids of the Aβ peptide selected from: ₂₂EDV₂₄, ₂₁AED_{23,} and/or ₂₃DVG_{25.}

6. An Aβ fragment-binding molecule according to any one of claims 1 to 5, wherein said Aβ fragment-binding molecule specifically binds to said neo-C-terminal fragment of Aβ peptide wherein the C-terminal amino acid is amidated.

7. An Aβ fragment-binding molecule according to claim 6, wherein said Aβ fragment-binding molecule specifically binds to a fragment of Aβ peptide that is Aβ1-24 of SEQ ID NO: 6 wherein the C-terminal Valine is amidated.

8. A pharmaceutical and/or diagnostic composition comprising one or more of: (i) an Aβ fragment-binding molecule according to any one of claims 1 to 7; (ii) a conjugate molecule comprising the Aβ fragment-binding molecule under (i); (iii) a nucleic acid encoding a molecule under (i) or polypeptidic conjugated molecule under (ii); (iv) a recombinant expression vector comprising the nucleic acid under (iii); and/or (v) a host cell comprising the recombinant expression vector under (iv), and at least one pharmaceutically acceptable carrier.

9. An *ex vivo* method of prognosis or diagnosis of a β-amyloid aggregation related disorder from a biological sample of a subject comprising the steps of:
(a) Providing a biological sample from a subject;
(b) Bringing said biological sample into contact with at least one Aβ fragment-binding molecule according to any one of claims 1 to 7, wherein the contacting is under conditions sufficient for binding at least one of said neo-C-terminal fragment of Aβ present in said biological fluid sample to said at least one Aβ fragment-binding molecule;
(c) Washing;
(d) Detecting a signal proportional to the level of complex formed in step (b) between said at least one neo-C-terminal fragment of Aβ and said at least one Aβ fragment-binding molecule,
(e) Comparing the level of signal detected in step (d) with the level of signal detected in the same conditions with a negative control,
wherein a level of signal detected in the subject's sample that is higher than the level of signal detected in the negative control is indicative of a β-amyloid aggregation related disorder.

10. An *ex vivo* method of claim 9 wherein the said biological sample is contacted with at least one further Aβ fragment-binding molecule specifically binding to a neo-N-terminal fragment obtained by cleavage of said Aβ peptide between two consecutive amino acids located between positions 20 and 26 on the amino acid sequence of said Aβ peptide, but which does not bind to Aβ peptides of 40, 42 and/or 43 amino acids (Aβ1-40, Aβ1-42, Aβ1-43), wherein said contacting is under conditions sufficient for binding at least one of said neo-N-terminal fragment of Aβ present in said biological fluid sample to said at least one further Aβ fragment-binding molecule.

11. An *ex-vivo* method according to claim 9 or 10 wherein said biological sample is a blood sample or a cerebrospinal fluid sample.

12. An isolated fragment of Aβ peptide, wherein said fragment is a neo-C-terminal fragment of Aβ peptide selected from: Aβ1-25, Aβ1-24, Aβ1-23, Aβ1-22, wherein C-terminal amino acid of said fragment is amidated.

13. A kit for use in a method for the detection of early stages of Aβ peptide aggregation and/or amyloid plaque formation comprising at least one Aβ fragment-binding molecule according to any one of claims 1 to 7, to be coupled or already coupled to a solid matrix.

## Patentansprüche

1. Aß-fragmentbindendes Molekül, **dadurch gekennzeichnet, dass** das Molekül ein Antikörper oder Fragment davon ist, der/das spezifisch an ein neo-C-terminales Fragment von Aß-Peptid bindet, das durch Spaltung des Aß-Peptids zwischen zwei aufeinanderfolgenden Aminosäuren erhalten wird, die sich zwischen den Positionen 20 und 26 auf der Aminosäuresequenz des Aß-Peptids befinden, aber nicht an Aß-Peptide von 40, 42 und/oder 43 Aminosäuren (Aßl-40, Aßl-42, Aßl-43) bindet.

2. Aß-fragmentbindendes Molekül nach Anspruch 1, wobei das neo-C-terminale Fragment des Aß-Peptids erhalten wird durch Spaltung des Aß-Peptids zwischen zwei aufeinanderfolgenden Aminosäuren, die sich an den Positionen 24 und 25, oder 23 und 24, oder 25 und 26, oder 22 und 23, auf der Aminosäuresequenz des Aß-Peptids befinden.

3. Aß-fragmentbindendes Molekül nach einem der Ansprüche 1 bis 2, wobei das neo-C-terminale Fragment Aßl-25 oder ein Teil davon ist, wobei 1 bis 3 Aminosäuren am C-terminalen Ende von Aßl-25 gelöscht werden.

4. Aß-fragmentbindendes Molekül nach Anspruch 3, wobei das neo-C-terminale Fragment ausgewählt wird aus: Aßl-24 und Aβ1-23.

5. Aß-fragmentbindendes Molekül nach einem der Ansprüche 1 bis 4, wobei das Aß-fragmentbindende Molekül spezifisch an das neo-C-terminale Fragment des Peptids Aβ bindet, indem es mit einem Epitop interagiert, das mindestens eine Aminosäure des Peptids Aβ umfasst, das ausgewählt wird aus: A21, E22, D23, V24, G25, oder drei aufeinanderfolgenden Aminosäuren des Peptids Aβ, ausgewählt aus: ₂₂EDV₂₄, ₂₁AED₂₃ und/oder ₂₃DVG₂₅.

6. Aß-fragmentbindendes Molekül nach einem der Ansprüche 1 bis 5, wobei das Aß-fragmentbindende Molekül spezifisch an das neo-C-terminale Fragment von Aß-Peptid bindet, wobei die C-terminale Aminosäure amidiert ist.

7. Aß-fragmentbindendes Molekül nach Anspruch 6, wobei das Aß-fragmentbindende Molekül spezifisch an ein Fragment von Aß-Peptid bindet, das Aßl-24 von SEQ ID NO: 6 ist, wobei das C-terminale Valin amidiert ist.

8. Pharmazeutische und/oder diagnostische Zusammensetzung, umfassend eine oder mehrere von: (i) ein Aß-fragmentbindendes Molekül nach einem der Ansprüche 1 bis 7; (ii) ein konjugiertes Molekül, das das Aß-fragmentbindende Molekül unter (i) umfasst; (iii) eine Nukleinsäure, die für ein Molekül unter (i) oder ein polypeptidisch konjugiertes Molekül unter (ii) kodiert; (iv) einen rekombinanten Expressionsvektor, der die Nukleinsäure unter (iii) umfasst; und/oder (v) eine Wirtszelle, umfassend den rekombinanten Expressionsvektor unter (iv), und mindestens einen pharmazeutisch akzeptablen Träger.

9. Ex-vivo-Verfahren zur Prognose oder Diagnose einer β-Amyloid-Aggregationsstörung aus einer biologischen Probe eines Patienten, umfassend die folgenden Schritte:
(a) Bereitstellen einer biologischen Probe von einem Patienten;
(b) Kontaktieren der biologischen Probe mit mindestens einem Aß-fragmentbindenden Molekül nach einem der Ansprüche 1 bis 7, wobei das Kontaktieren unter Bedingungen erfolgt, die ausreichen, um mindestens eines der in der biologischen Flüssigkeitsprobe vorhandenen neo-C-terminalen Fragmente von Aβ an das mindestens eine Aß-fragmentbindende Molekül zu binden;
(c) Waschen;
(d) Erfassen eines Signals, das proportional zum Pegel des Komplexes ist, der in Schritt (b) zwischen dem mindestens einen neo-C-terminalen Fragment von Aβ und dem mindestens einen Aß-fragmentbindenden Molekül gebildet wird,
(e) Vergleichen des in Schritt (d) erfassten Signalpegels mit dem unter den gleichen Bedingungen erfassten Signalpegel mit einer negativen Kontrolle,
wobei ein in der Probe des Patienten erfassten Signalpegel, der höher ist als der in der negativen Kontrolle erfassten Signalpegel, auf eine β-Amyloidaggregationsbezogene Störung hinweist.

10. *Ex vivo*-Verfahren nach Anspruch 9, wobei die biologische Probe mit mindestens einem weiteren Aß-fragmentbindenden Molekül in Kontakt gebracht wird, das spezifisch an ein neo-N-terminales Fragment bindet, das durch Spaltung des Aß-Peptids zwischen zwei aufeinanderfolgenden Aminosäuren erhalten wird, die sich zwischen den Positionen 20 und 26 auf der Aminosäuresequenz des Aß-Peptids befinden, die aber nicht an Aß-Peptide von 40, 42 und/oder 43 Aminosäuren (Aßl-40, Aßl-42, Aßl-43) bindet, wobei das Kontaktieren unter Bedingungen erfolgt, die ausreichen, um mindestens eines der in der biologischen Flüssigkeitsprobe vorhandenen neo-N-terminalen Fragmente von Aβ an das mindestens eine weitere Aß-fragmentbindende Molekül zu binden.

11. *Ex-vivo*-Verfahren nach Anspruch 9 oder 10, wobei die biologische Probe eine Blutprobe oder eine Liquorprobe ist.

12. Isoliertes Fragment von Aβ-Peptid, wobei das Fragment ein neo-C-terminales Fragment von Aβ-Peptid ist, ausgewählt aus: Aßl-25, Aβ1-24, Aβ1-23, Aβ1-23, Aβ1-22, wobei die C-terminale Aminosäure des Fragments amidiert ist.

13. Kit zur Verwendung in einem Verfahren zum Nachweis von Frühstadien der Aß-Peptidaggregation und/oder Amyloid-Plaquebildung, das mindestens ein Aß-fragmentbindendes Molekül nach einem der Ansprüche 1 bis 7 umfasst, das an eine feste Matrix gekoppelt sein soll oder bereits gekoppelt ist.

## Revendications

1. Molécule se liant au fragment Aβ **caractérisée en ce que** ladite molécule est un anticorps ou un fragment de celui-ci qui se lie spécifiquement à un néo-fragment C-terminal du peptide Aβ obtenu par clivage dudit peptide Aβ entre deux acides aminés consécutifs situés entre les positions 20 et 26 sur la séquence d'acides aminés dudit peptide Aβ, mais ne se lie pas aux peptides Aβ de 40, 42 et/ou 43 acides aminés (Aβ1-40, Aβ1-42, Aβ1-43).

2. Molécule se liant au fragment Aβ selon la revendication 1, dans laquelle ledit néo-fragment C-terminal du peptide Aβ est obtenu par clivage dudit peptide Aβ entre deux acides aminés consécutifs situés aux positions 24 et 25, ou 23 et 24, ou 25 et 26, ou 22 et 23, sur la séquence d'acides aminés dudit peptide Aβ.

3. Molécule se liant au fragment Aβ selon l'une quelconque des revendications 1 à 2, dans laquelle ledit néo-fragment C-terminal est Aβ1-25 ou une partie de celui-ci où 1 à 3 acides aminés à l'extrémité C-terminale du Aβ1-25 sont délétés.

4. Molécule se liant au fragment Aβ selon la revendication 3, dans laquelle ledit néo-fragment C-terminal est choisi parmi : Aβ1-24 et Aβ1-23.

5. Molécule se liant au fragment Aβ selon l'une quelconque des revendications 1 à 4, dans laquelle ladite molécule se liant au fragment Aβ se lie spécifiquement audit néo-fragment C-terminal du peptide Aβ par interaction avec un épitope comprenant au moins un acide aminé du peptide Aβ choisi parmi : A21, E22, D23, V24, G25, ou trois acides aminés consécutifs du peptide Aβ choisis parmi : ₂₂EDV₂₄, ₂₁AED₂₃ et/ou ₂₃DVG₂₅.

6. Molécule se liant au fragment Aβ selon l'une quelconque des revendications 1 à 5, dans laquelle ladite molécule se liant au fragment Aβ se lie spécifiquement audit néo-fragment C-terminal du peptide Aβ dans lequel l'acide aminé C-terminal est amidé.

7. Molécule se liant au fragment Aβ selon la revendication 6, dans laquelle ladite molécule se liant au fragment Aβ se lie spécifiquement à un fragment du peptide Aβ qui est Aβ1-24 de SEQ ID No: 6 dans lequel la valine C-terminale est amidée.

8. Composition pharmaceutique et/ou diagnostique comprenant un ou plusieurs de : (i) une molécule se liant au fragment Aβ selon l'une quelconque des revendications 1 à 7 ; (ii) une molécule conjuguée comprenant la molécule se liant au fragment Aβ en (i) ; (iii) un acide nucléique codant pour la molécule en (i) ou la molécule conjuguée polypeptidique en (ii) ; (iv) un vecteur d'expression recombiné comprenant l'acide nucléique en (iii) ; et/ou (v) une cellule hôte comprenant le vecteur d'expression recombiné en (iv), et au moins un véhicule pharmaceutiquement acceptable.

9. Méthode *ex vivo* de pronostic ou de diagnostic d'un trouble lié à l'agrégation de la β-amyloïde à partir d'un échantillon biologique d'un sujet comprenant les étapes consistant à :
(a) Se procurer un échantillon biologique d'un sujet ;
(b) Amener ledit échantillon biologique en contact avec au moins une molécule se liant au fragment Aβ selon l'une quelconque des revendications 1 à 7, où la mise en contact s'effectue dans des conditions suffisantes pour lier au moins l'un desdits néo-fragments C-terminaux de Aβ présents dans ledit échantillon de fluide biologique à ladite au moins molécule se liant au fragment Aβ ;
(c) Laver ;
(d) Détecter un signal proportionnel au niveau du complexe formé à l'étape (b) entre ledit au moins néo-fragment C-terminal de Aβ et ladite au moins molécule se liant au fragment Aβ,
(e) Comparer le niveau de signal détecté à l'étape (d) avec le niveau de signal détecté dans les mêmes conditions avec un témoin négatif,
dans laquelle un niveau de signal détecté dans l'échantillon du sujet qui est supérieur au niveau de signal détecté dans le témoin négatif indique un trouble lié à l'agrégation de la β-amyloïde.

10. Méthode *ex vivo* selon la revendication 9 dans laquelle ledit échantillon biologique est mis en contact avec au moins une autre molécule se liant au fragment Aβ qui se lie spécifiquement à un néo-fragment C-terminal obtenu par clivage dudit peptide Aβ entre deux acides aminés consécutifs situés entre les positions 20 et 26 sur la séquence d'acides aminés dudit peptide Aβ, mais ne se lie pas aux peptides Aβ de 40, 42 et/ou 43 acides aminés (Aβ1-40, Aβ1-42, Aβ1-43), où ladite mise en contact s'effectue dans des conditions suffisantes pour lier au moins l'un desdits néo-fragments C-terminaux de Aβ présents dans ledit échantillon de fluide biologique à ladite au moins autre molécule se liant au fragment Aβ.

11. Méthode *ex vivo* selon la revendication 9 ou 10 dans laquelle ledit échantillon biologique est un échantillon de sang ou un échantillon de fluide céphalorachidien.

12. Fragment isolé de peptide Aβ, dans lequel ledit fragment est un néo-fragment C-terminal du peptide Aβ choisi parmi : Aβ1-25, Aβ1-24, Aβ1-23, Aβ1-22, dans lequel l'acide aminé C-terminal dudit fragment est amidé.

13. Kit destiné à être utilisé dans une méthode de dépistage des stades précoces de l'agrégation du peptide Aβ et/ou de la formation de plaques amyloïdes comprenant au moins une molécule se liant au fragment Aβ selon l'une quelconque des revendications 1 à 7, à coupler ou déjà couplée à une matrice solide.


### SEQUENCE LISTING

<110> ECOLE POLYTECHNIQUE FEDERALE DE LAUSANNE (EPFL)
<120> AGENTS USEFUL IN DIAGNOSTIC AND THERAPY OF BETA-AMYLOID AGGREGATION RELATED DISORDERS AND SCREENING METHODS
<130> P1790PC00
<150> EP14162767
   <151> 2014-03-31
<160> 26
<170> PatentIn version 3.5
<210> 1
   <211> 42
   <212> PRT
   <213> Homo sapiens
<400> 1
<210> 2
   <211> 40
   <212> PRT
   <213> Homo sapiens
<400> 2
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val
   35 40
<210> 3
   <211> 43
   <212> PRT
   <213> Homo sapiens
<400> 3
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala Thr
   35 40
<210> 4
   <211> 22
   <212> PRT
   <213> Homo sapiens
<400> 4
   Asp Ala Glu Phe Arg His Asp Ser Gly Tyr Glu Val His His Gln Lys
   1 5 10 15
   Leu Val Phe Phe Ala Glu
   20
<210> 5
   <211> 23
   <212> PRT
   <213> Homo sapiens
<400> 5
   Asp Ala Glu Phe Arg His Asp Ser Gly Tyr Glu Val His His Gln Lys
   1 5 10 15
   Leu Val Phe Phe Ala Glu Asp
   20
<210> 6
   <211> 24
   <212> PRT
   <213> Homo sapiens
<400> 6
   Asp Ala Glu Phe Arg His Asp Ser Gly Tyr Glu Val His His Gln Lys
   1               5                   10                  15
   Leu Val Phe Phe Ala Glu Asp Val
               20
<210> 7
   <211> 25
   <212> PRT
   <213> Homo sapiens
<400> 7
   Asp Ala Glu Phe Arg His Asp Ser Gly Tyr Glu Val His His Gln Lys
   1               5                   10                  15
   Leu Val Phe Phe Ala Glu Asp Val Gly
               20                  25
<210> 8
   <211> 13
   <212> PRT
   <213> Homo sapiens
<400> 8
   Asp Ala Glu Phe Arg His Asp Ser Gly Tyr Glu Val His
   1               5                   10
<210> 9
   <211> 14
   <212> PRT
   <213> Homo sapiens
<400> 9
   Asp Ala Glu Phe Arg His Asp Ser Gly Tyr Glu Val His His
   1               5                   10
<210> 10
   <211> 15
   <212> PRT
   <213> Homo sapiens
<400> 10
   Asp Ala Glu Phe Arg His Asp Ser Gly Tyr Glu Val His His Gln
   1               5                   10                  15
<210> 11
   <211> 18
   <212> PRT
   <213> Homo sapiens
<400> 11
   Asp Val Gly Ser Asn Lys Gly Ala Ile Ile Gly Leu Met Val Gly Gly
   1 5 10 15
   Val Val
<210> 12
   <211> 19
   <212> PRT
   <213> Homo sapiens
<400> 12
   Val Gly Ser Asn Lys Gly Ala Ile Ile Gly Leu Met Val Gly Gly Val
   1 5 10 15
   Val Ile Ala
<210> 13
   <211> 18
   <212> PRT
   <213> Homo sapiens
<400> 13
   Gly Ser Asn Lys Gly Ala Ile Ile Gly Leu Met Val Gly Gly Val Val
   1 5 10 15
   Ile Ala
<210> 14
   <211> 17
   <212> PRT
   <213> Homo sapiens
<400> 14
   Ser Asn Lys Gly Ala Ile Ile Gly Leu Met Val Gly Gly Val Val Ile
   1 5 10 15
   Ala
<210> 15
   <211> 18
   <212> PRT
   <213> Homo sapiens
<400> 15
   Asp Val Gly Ser Asn Lys Gly Ala Ile Ile Gly Leu Met Val Gly Gly
   1 5 10 15
   Val Val
<210> 16
   <211> 17
   <212> PRT
   <213> Homo sapiens
<400> 16
   Val Gly Ser Asn Lys Gly Ala Ile Ile Gly Leu Met Val Gly Gly Val
   1 5 10 15
   Val
<210> 17
   <211> 16
   <212> PRT
   <213> Homo sapiens
<400> 17
   Gly Ser Asn Lys Gly Ala Ile Ile Gly Leu Met Val Gly Gly Val Val
   1 5 10 15
<210> 18
   <211> 15
   <212> PRT
   <213> Homo sapiens
<400> 18
   Ser Asn Lys Gly Ala Ile Ile Gly Leu Met Val Gly Gly Val Val
   1 5 10 15
<210> 19
   <211> 21
   <212> PRT
   <213> Homo sapiens
<400> 19
   Asp Val Gly Ser Asn Lys Gly Ala Ile Ile Gly Leu Met Val Gly Gly
   1 5 10 15
   Val Val Ile Ala Thr
   20
<210> 20
   <211> 20
   <212> PRT
   <213> Homo sapiens
<400> 20
   Val Gly Ser Asn Lys Gly Ala Ile Ile Gly Leu Met Val Gly Gly Val
   1               5                   10                  15
   Val Ile Ala Thr
               20
<210> 21
   <211> 19
   <212> PRT
   <213> Homo sapiens
<400> 21
   Gly Ser Asn Lys Gly Ala Ile Ile Gly Leu Met Val Gly Gly Val Val
   1               5                   10                  15
   Ile Ala Thr
<210> 22
   <211> 18
   <212> PRT
   <213> Homo sapiens
<400> 22
   Ser Asn Lys Gly Ala Ile Ile Gly Leu Met Val Gly Gly Val Val Ile
   1               5                   10                  15
   Ala Thr
<210> 23
   <211> 12
   <212> PRT
   <213> Homo sapiens
<400> 23
   His Gln Lys Leu Val Phe Phe Ala Glu Asp Val Gly
   1               5                   10
<210> 24
   <211> 11
   <212> PRT
   <213> Homo sapiens
<400> 24
   Gln Lys Leu Val Phe Phe Ala Glu Asp Val Gly
   1               5                   10
<210> 25
   <211> 10
   <212> PRT
   <213> Homo sapiens
<400> 25
   Lys Leu Val Phe Phe Ala Glu Asp Val Gly
   1               5                   10
<210> 26
   <211> 43
   <212> PRT
   <213> Mus musculus
<400> 26
   Asp Ala Glu Phe Gly His Asp Ser Gly Phe Glu Val Arg His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40